(19) Europäisches Patentamt
European Patent Office
Office européen des brevets

(11) Veröffentlichungsnummer: **0 455 769 B1**

# (12) EUROPÄISCHE PATENTSCHRIFT

(45) Veröffentlichungstag der Patentschrift: **27.09.95**

(51) Int. Cl.[6]: **A61K 31/37**

(21) Anmeldenummer: **90917032.6**

(22) Anmeldetag: **23.11.90**

(86) Internationale Anmeldenummer:
**PCT/EP90/02003**

(87) Internationale Veröffentlichungsnummer:
**WO 91/07959 (13.06.91 91/13)**

## (54) VERWENDUNG VON 7-OH-1,2-BENZOPYRON ZUR HERSTELLUNG EINES ARZNEIMITTELS ZUR BEHANDLUNG VON MALIGNEN TUMOREN BEIM MENSCHEN.

(30) Priorität: **24.11.89 DE 3938902**

(43) Veröffentlichungstag der Anmeldung:
**13.11.91 Patentblatt 91/46**

(45) Bekanntmachung des Hinweises auf die Patenterteilung:
**27.09.95 Patentblatt 95/39**

(84) Benannte Vertragsstaaten:
**AT BE CH DE DK FR GB IT LI LU NL SE**

(56) Entgegenhaltungen:

**Journal of Natural Products, Bd 46, 248 - 250 (1983)**

**Cancer Chemotherapy Reports Teil 3, Bd 3 (2) (1972)**

**Journal of Cellular Biochemistry, Bd 36, 353 - 367 (1988)**

(73) Patentinhaber: **SCHAPER & BRÜMMER GMBH & CO. KG**
**Bahnhofstrasse 45**
**D-38259 Salzgitter (DE)**

(72) Erfinder: **THORNES, R., Douglas**
**28 Offington Road**
**Dublin 13 (IE)**
Erfinder: **STOLZE, Helga**
**Nonnenstieg 86**
**D-3400 Göttingen (DE)**
Erfinder: **MARSHALL, Ernest**
**2885 Ark Royal Way**
**Lexington, KY 40503 (US)**
Erfinder: **ZÄNKER, Kurt**
**Agnes-Bernauer-Str. 131**
**D-8000 München 21 (DE)**

(74) Vertreter: **UEXKÜLL & STOLBERG Patentanwälte**
**Beselerstrasse 4**
**D-22607 Hamburg (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

Römpp Chemielexikon, 9. Auflage, 2071, 2998

Immunology Today, Bd 9, 157 - 159 (1988)

Planta Medica, Band 53, Nr. 6, 1987, A. Gawron et al.: "Cytostatic activity of coumarins in vitro", Seiten 526-529

Chemical Abstract, Band 99, Nr. 1, 4. Juli 1983, (Columbus, Ohio, US), S.S. Handa et l.: "Plant antivancer agents. XXVI. Constituents of Peddiea fischeri", Seite 301, Abstract 3057d

Chemical Abstracts, vol. 105, No. 25, 22 December 1986, (Columbus, Ohio, US), C.Y. Duh et al.pages 448-449, abstract 222731q

Pharmazie, vol 36, H. 2,1981, M.A. Loutfy: Page 166

Cancer Research, vol 39, May 1979, L.W. Wattenberg et al. Pages 1651-1654, abstract; page 1652

Journal of the National Cancer Institute, vol. 56, No. 6, June 1976, B.L. Van Duuren et al., pages 1237-1242, abstract; page 1239

J. Neurosurg. vol. 71, October 1989, D.F. Wilson et al. pages 551-557, abstract ; page 555, "table 3"

Chemical Abstracts, vol. 104, No. 9,3 March 1986, (Columbus, Ohio US)R. Kato et al. page 28, abstract 61607r

Dialog Information Services , File 5, Biosis No. 83035714, Accession No.5773407, K.S. Kang et al.

## Beschreibung

Die Erfindung betrifft die Verwendung von 7-OH-1,2-Benzopyron (7-OH-1,2-BP) zur Herstellung von Arzneimitteln zur präventiven und therapeutischen Behandlung maligner Tumore beim Menschen.

Die gegenwärtig zur Verfügung stehenden Konzepte zur onkologischen Therapie beinhalten die Therapie mit Substanzen oder Strahlen, um dadurch einen unmittelbaren Zelltod und mit diesem Reduktion der Volumina von Primärtumor und/oder Metastasen zu erreichen. Für strahlensensible Tumore kann dieses Prinzip lokal günstig durchgehalten werden, während die Therapie mit cytostatischen/cytotoxischenSubstanzen, wenn nicht die loko-regionale Applikation technisch möglich ist, immer systemisch auf den gesamten Organismus wirkt. Das Konzept der systemischen Cytostase/Cytotoxizität ist in seiner biologischen Effizienz durch das Spektrum der aktuellen Nebenwirkungen eingeschränkt. So hat man beispielsweise versucht, durch Modifikation von cytotoxischen Molekülen, wie cis-Platin zu Carbo-Platin, toxische Nebenwirkungen auf ein tolerierbares Maß zu senken. Ein weiteres Modifikationsprinzip ist der Zusatz von Substanzen, wie Hyaluronidase, Verapramil o.a., welche die biologische Wirksamkeit steigern, um, mit dem Ziel einer Verringerung der Nebenwirkungen, auf diese Weise mit einer niedrigeren Dosierung des jeweiligen Cytostatikums auszukommen. Dieses Konzept ist in einzelnen Protokollen erfolgreich zum Einsatz gekommen.

Es ist ferner versucht worden, Substanzen einzusetzen, die noch andere Wirkmechanismen als die direkte Cytostase aufweisen, bzw. die neben der Cytostase über andere Mechanismen verfügen. So ist es von Cyclophosphamid bekannt, daß es in niedrigen Konzentrationen die T4-Lymphozyten in ihrer biologischen Aktvitität inhibiert. Es kann somit eine Immunmodulation mit einem Cytostatikum erreicht werden.

Neue Therapieansätze in der Onkologie haben sich insbesondere auf Natursubstanzen konzentriert, die als Reinsubstanzen entweder durch pflanzenextraktion oder durch chemische Synsthese darstellbar sind. So zeigte es sich, daß Cumarin in Zellkulturen und in Tierexperimenten eine Inhibition von malignem Zellwachstum bewirkt, wobei empirisch gefundene Nebenwirkungen bei Säugern im Vergleich zur biologischen Aktivität zu vernachlässigen sind (vgl. beispielsweise K.S. Zänker, et al.: "Coumarin in melanoma patients, an experimental and clinical study", Drugs Exptl. Clin. Res. X (11) 767-774 (1984); M.E. Marshall et al., "Treatment of metastatic renal cell carcinoma with Coumarin (1,2-Benzopyrone) and Cimetidine, A pilot study", Journal of Clinical Oncology, 5, 862-866 (1987); D. Thornes et al., "Prevention of early recurrence of high risk malignant melanoma by coumarin" European Journal of Surgical Oncology 15, 431-435 (1989)).

In vitro Untersuchungen an HELA-Zellen sind in Plantamedica 53, 526 - 529 (1987) beschrieben. Sie wurden sowohl an Gesamt-Cumarin-Fraktionen aus Angelica und Pastinak durchgeführt als auch an Einzelfraktionen nach Auftrennung der Gesamtextrakte. Dabei zeigte es sich, daß unter den "einfachen Cumarinen" nur Osthol und 4-Methyl-Aesculetin signifikante Hemmwirkung auf die Kulturen ausübten. Starke Wirkung wurde jedoch bei den Furocumarinen gefunden.

In Journal of Natural Products 46, 248 - 250 (1983) sind Untersuchungen beschrieben, bei denen die cytostatische Wirksamkeit verschiedener Cumarinderivate an einer aus Mäusen stammenden Zellinie untersucht werden (P-388 Testsystem). Die Ergebnisse dieser Untersuchungen zeigen, daß Daphnoterin und Umbifelleron keinerlei in vivo biologische Aktivität zeigen und daher bei der Maus als Cytostatikum ungeeignet sind.

In Pharmazie, 36, H2, 166 (1986) wird die Herstellung und die Untersuchung von Antitumoreigenschaften bestimmter Cumarine und Psoralene, u.a. von 7-OH-1,2-Benzopyron beschrieben. Die Verbindungen wurden für Antitumor-Untersuchungen an das National Cancer Institute (USA) gegeben und es wurde gefunden, daß keine der Substanzen bei den untersuchten Dosierungen Antitumoraktivität entfaltete.

Schließlich sind in Cancer Research 39, 1651 - 1654 (1979) Untersuchungen beschrieben, im Rahmen derer die cytostatische Wirksamkeit von 7-OH-Benzopyron an chemisch induzierten Mammakarzinomen der Ratte untersucht wurden. In diesen Versuchen erwies sich die Substanz als wirkungslos.

Es ist Aufgabe der vorliegenden Erfindung, Substanzen zur präventiven und therapeutischen Behandlung von malignen Tumoren beim Menschen auf der Basis von Natursubstanzen zur Verfügung zu stellen, welche bezüglich ihrer biologischen Aktivität den bereits erprobten Substanzen überlegen und die gleichzeitig toxikologisch unbedenklich sind.

Zur Lösung der Aufgabe wird die Verwendung von 7-OH-1,2-Benzopyron vorgeschlagen.

Das erfindungsgemäß verwendete 7-OH-1,2-Benzopyron kann nach bekannten Verfahren hergestellt werden, vgl. Beilstein, E III/IV 18, 294 ff.

Schließlich kann die erfindungsgemäße Substanz nach dem Fachmann bekannten Verfahren enzymatisch im Fermenter hergestellt werden.

Überraschenderweise hat es sich gezeigt, daß 7-Hydroxy-1,2-Benzopyron eine unerwartet hohe in vivo Aktivität im Hinblick auf die Hemmung des Wachstums von Tumorzellen, die Rückbildung von Tumoren oder die Hemmung von Metastasen entfalten.

Wie die Tabellen 1 bis 3 zeigen, konnte die wachstumshemmende Wirkung der erfindungsgemäßen Substanz in vivo an zahlreichen Tumorzellen unterschiedlichen Ursprungs nachgewiesen werden.

Tabelle 1

| Zellinien (human) | | Konz. 7-OH-1,2-BP im Wachstumsmedium | % Wachstumshemmung gegenüber Kontrolle nach x Tagen |
|---|---|---|---|
| Ursprung | Kennzeichen | µMol/ml | |
| Prostata-Karzinom | LNCap | 250 | 66 x = 10 |
| anaplastisches Astrozytom | g-CCM | 200 | 44 x = 10 |
| anaplastisches Astrozytom | g-UVW | 200 | 40 x = 10 |
| Brust-Karzinom | MCF 7 | 200 | 64 x = 10 |
| Blasen-Karzinom | EJ | 200 | 26 x = 10 |
| Burkitt-Lymphom (Leukämie) | Daudi | 1,2 | 94,8 x = 9 |
| Glioblastom | U 178 MG | 25 | 74 x = 12 |
| Neuroblastom | TP 410 N | 25 | 62 x = 12 |
| Glioblastom | TP 242 MG | 25 | 79 x = 12 |
| Glioblastom | TP 336 MG | 10 | 60 x = 12 |
| epidermoides Karzinom | A 431 | 10 | 76 x = 12 |

Es ist von besonderer Bedeutung, daß 7-Hydroxy-1,2-Benzopyron starke wachstumshemmende Wirkung auf Gehirntumorzellen (Glioblastomzellen) ausübt (vgl. Tabellen 1 bis 3), da bisher weder Chemotherapie noch Bestrahlungsbehandlung oder die Therapie mit sogenannten BRM (Biological Response Modifiern) zu therapeutischen Erfolgen führte. Abgesehen von den Interferonen konnte eine in vivo Proliferationshemmung von Gehirntumorzellen erstmals mit der erfindungsgemäßen Substanz gezeigt werden.

Im Zusammenhang mit der neoplastischen Transformation und erhöhten Proliferationsrate von Glioblastomzellen werden zwei autokrine Loops, das EGF- und das PDGF-System diskutiert. Es konnte gezeigt werden, daß durch 7-OH-1,2-BP Veränderungen auf der Ebene der Genexpression eines dieser Systeme eintreten. So wurde nachgewiesen (Seliger et al., unveröffentlichte Ergebnisse), daß die Transkription der für PDFG-A und PDFG-B kodierenden Gene inhibiert wird (die entsprechenden mRNA-Spiegel sinken, vgl. Figur 8), während die Expression der PDGF-Rezeptoren und des EGF-Systems nicht beeinflußt wird. Es ist denkbar, daß die durch 7-OH-1,2-BP hervorgerufene Inhibition der PDFG-mRNA für die beobachtete Wachtumshemmung verantwortlich ist, indem ein autokriner Loop unterbrochen wird.

Besondere Eignung weist 7-OH-1,2-Benzopyron nach der Erfindung ferner zur Behandlung von Nieren-, Blasen-, Prostata-, Haut- oder Lungenkarzinomen sowie von Leukämie auf.

Die erfindungsgemäße Substanz kann sowohl allein als auch in Kombination mit herkömmlichen Therapeutika und mit sonstigen Verfahren zur Behandlung maligner Tumore eingesetzt werden.

So haben sich durch die Kombination des erfindungsgemäßen 7-OH-1,2-Benzopyrons mit Chemotherapeutika wie cis-Platin und 5-Fluorouracil (5-FU) unerwartete synergistische Effekte gezeigt (vgl. Beispiel 6).

Die erfindungsgemäße Substanz kann ferner zusätzlich zur herkömmlichen Therapie mit Cytokinen und Monokinen sowie neben einer Strahlentherapie eingesetzt werden, um deren Wirksamkeit zu erhöhen und die Toxizität des Therapieschemas zu senken. Es konnte an verschiedenen Zellinien aus Prostatakarzinomen gezeigt werden, daß die Verwendung einer Kombination von 7-OH-1,2-BP mit Tumor-Nekrose-Faktor (TFN) zu einer überadditiven Hemmung des Zellwachstums führt (vgl. Beispiel 7).

Ferner wurde gefunden, daß die bei hormonabhängigen Tumorzellen, wie beispielsweise (LNCaP-Zellen), die durch 7-OH-1,2-BP bewirkte Wachtumshemmung durch zusätzliche Hormontherapie im herkömmlichen Sinne, beispielsweise mit Testosteron, gesteigert werden kann (vgl. Beispiel 8).

Die Verwendung der erfindungsgemäßen Substanz kommt sowohl zur Prävention maligner Tumore als auch zur akuten und adjuvanten Therapie in Betracht.

Bei der präventiven Behandlung mit der erfindungsgemäßen Substanz spielt insbesondere deren Fähigkeit zur Chemoprävention sowie zur Hemmung der Onkogenexpression eine wichtige Rolle. Träger von Onkogenen unterliegen einem erhöhten Krebsrisiko, da diese Gene auf vielfältige Weise aktiviert werden und die Expressionsprodukte die Bildung von Tumoren induzieren können. Der Begriff "Chemoprävention" bezeichnet die Fähigkeit geeigneter Substanzen, die durch Onkogenexpression induzierte Tumorbildung zu verhindern oder zu verzögern. Das Modell der transgenen Maus bietet eine besondere Möglichkeit, die chemopräventive Wirkung von Testsubstanzen zu überprüfen. (Dem fertilen Oozyten bestimmter Mäusestämme werden durch Genmanipulation ein Onkogen - MTV/ras - tranferiert und der Oozyt reimplantiert und ausgetragen. Das ausgewachsene Tier und dessen Nachkommen tragen das Onkogen in den somatischen Zellen). Die transgenen Mäuse entwickeln mit hoher Frequenz Tumore. Erfindungsgemäß konnte gezeigt werden, daß bei diesen Tieren durch permanente Behandlung mit 7-OH-1,2-BP die Tumorinzidenz gegenüber den Kontrolltieren signifikant gesenkt werden konnte (vgl. Beispiel 11).

In diesem Zusammenhang ist auch bekannt, daß die Überlebenszeit von Patienten, deren Tumore bestimmte Onkogene, wie "c-myc" und "H-ras" überproportional exprimieren, geringer ist als die Überlebenszeit von Tumorpatienten, deren Geschwulst keine erhöhte Onkogenexpression aufweist. Erfindungsgemäß konnte nun nachgewiesen werden, daß 7-OH-1,2-BP das Zellwachstum von Tumorzellen mit Onkogen-Überexpression hemmt (vgl. Beispiel 12).

Ferner hat es sich gezeigt, daß 7-OH-1,2-Benzopyron in niedrigen Dosismengen immunmodulierende Wirksamkeit aufweist (vgl. Beispiel 10 und Figuren 5 bis 7). Insbesondere konnte an humanen mononukleären Zellen (MNC) nachgewiesen werden, daß die Stimulation der Zellen mit Kombinationen aus 7-OH-BP und Endotoxinen wie Lipopolysacchariden bakteriellen Ursprungs eine vernetzte Interaktion verschiedener Cytokine zur Folge hat. Wie Figur 7 zeigt, führt eine 10-fache Erhöhung des Il-1 Spiegels zu einer 7-fachen Erhöhung des TNF-(Tumor-Nekrose-Faktor)Spiegels. Ähnliche Ergebnisse wurden für Il-6 erhalten, wobei jedoch eine 10-fache Erhöhung des Il-6 Spiegels mit einer 4-fachen Erhöhung des TNF-Spiegels einhergeht.

Wird die erfindundungsgemäße Substanz im oben erläuterten Sinne zur präventiven Behandlung maligner Tumore eingesetzt, so kommen Dosismengen von beispielsweise 50 bis 300 mg je Patient pro Tag in Betracht.

Bei der therapeutischen Behandlung maligner Tumoren liegt der Schwerpunkt auf den wachstumshemmenden Eigenschaften der erfindungsgemäßen Substanz, die bei höherer Dosierung von beispielsweise 300 bis 6000 mg pro Tag zum Tragen kommen. Entsprechendes gilt für die Behandlung nach Primärtherapie, um ein Tumorrezidiv und Metastasenbildung zu verhindern.

Von besonderer Bedeutung - insbesondere im Hinblick auf die bisher üblichen Therapien maligner Tumoren - ist die Tatsache, daß bei der Anwendung der erfindungsgemäßen Substanz am Menschen selbst bei Langzeitapplikation von extrem hohen Dosismengen (7000 mg/Tag, vgl. Beispiel 9) keinerlei toxische Nebenwirkungen beobachtet werden konnten.

Die erfindungsgemäß verwendete Substanz ist demgemäß im Sinne der Erfindung gleichzeitig hoch wirksam und untoxisch.

Wie oben dargelegt, kann das erfindungsgemäß verwendete 7-OH-1,2-Benzopyron allein oder in Kombination, vorzugsweise als fixe Kombination, mit bekannten Cytostatika und/oder Cytokinen appliziert werden. Bei alleiniger Verabreichung liegen die Tagesdosismengen abhängig von der Indikation und therapeutischen Zielsetzung zwischen 50 und 300 mg, wenn die immunmodulierende Wirkung im Vordergrund stehen soll und zwischen 300 und 6000 mg, wenn der Schwerpunkt auf dem cytostatischen Aspekt liegt.

Diese Dosismengen können in Form der üblichen pharmazeutischen Darreichungsformen, wie beispielsweise als Lösungen, Dragees, Kapseln, Tabletten, Injektions- oder Infusionslösungen, oral oder parenteral, wie beispielsweise intramuskulär, intraarteriell, intravenös oder auch topisch wie beispielsweise in Form von transdermalen Pflastern appliziert werden.

Für die Applikation eignen sich sterile wäßrige Lösungen, die den erfindungsgemäß vorgesehenen Wirkstoff enthalten. Diese Lösungen können erforderlichenfalls in geeigneter Weise gepuffert sein; das flüssige Verdünnungsmittel kann außerdem mit ausreichend Salzlösung oder Glukose isotonisch eingestellt sein.

Zur Herstellung der Arzneimittel auf Basis des erfindungsgemäß verwendeten 1,2-Benzopyron-Derivats können die üblichen Träger- und Zusatzstoffe verwendet werden. Übliche Trägerstoffe sind z.B. Wasser, physiologische Kochsalzlösung, Alkohole, Polyethylenglykole, Glycerinester, Gelatine, Kohlenhydrate, wie Laktose und Stärke, Calciumcarbonat, Magnesiumstearat, Talkum. Übliche Zusatzstoffe sind z.B. Konservierungsstoffe, Gleitmittel, Netzmittel und Emulgatoren, Farbstoffe, Geschmackskorrigentien und Aromastoffe. Die Auswahl der Träger- und Zusatzstoffe hängt davon ab, ob die erfindungsgemäßen Zubereitungen enteral, parenteral oder lokal appliziert werden sollen.

Die Erfindung wird nachfolgend anhand von Beispielen erläutert:

**Beispiel 1**

**Wachstumshemmende Wirkung von 7-Hydroxy-1,2-Benzopyron auf verschiedene Tumorzellinien - Vergleich mit Cumarin**

Die folgenden Zellinien wurden untersucht:

| | |
|---|---|
| Glioblastom | U 178 MG |
| Neuroblastom | TP 410 N |
| Glioblastom | TP 242 Mg |
| Glioblastom | TP 336 MG |
| Epidermoides Karzinom | A 431 |

Die Neuroblastom-Zellinie und die drei Glioblastom-Zellinien wurden in Hams F10-Medium angereichert mit 10% fötalem Kälberserum (FCS) sowie der üblichen Menge an Penicillin-Streptomycin (Pen/Strep) und Glutamin, kultiviert. Die epidermoide KarzinomZellinie wurde in modifiziertem Eagle's Medium (MEM), ebenfalls angereichert mit 10% (FCS) und der üblichen Menge an Glutamin und Pen/Strep kultiviert.

In einem Vorversuch wurden zunächst jeweils 5 x $10^3$ Zellen jeder Linie in 96-Loch-Platten ausplattiert und mit unterschiedlichen Konzentrationen, nämlich 150 µMol, 75 µMol, 37,5 µMol, 18 µMol und 9 µMol je ml Medium 7-Hydroxy-1,2-benzopyron 48 Stunden lang inkubiert. Als Kontrolle dienten unbehandelte Zellen. Anschließend wurden die Zellen 8 Stunden lang mit $^3$H-Thymidin inkubiert. Die in die Zellen aufgenommene Radioaktivität wurde auf übliche Weise gemessen. Es zeigte sich, daß die Linien TP 336 und A 431 sensitiver gegenüber (7-OH-1,2-BP) waren, als U 178, TP 242 und TP 410 N. Die Konzentration 37,5 µM/ml des Wirkstoffes war jedoch für keine der Zellinien cytotoxisch sondern cytostatisch.

Auf der Basis dieser Ergebnisse wurde für die genannten Zellinien eine Wachstumskurve über einen Zeitraum von insgesamt 12 Tagen erstellt, wobei die Linien TP 336 MG und A 431 unter den oben angegebenen Bedingungen mit jeweils 10 µM/ml 7-OH-1,2-BP inkubiert wurden, während bei den Linien TP 242 MG, TP 410 N und U 178 MG 25 µM/ml des Wirkstoffes verwendet wurden. Es wurden jeweils 5 x $10^4$ Zellen in $T_{25}$er Flaschen ausplattiert, wobei unbehandelte Zellen unter analogen Bedingungen als Kontrolle inkubiert wurden. Die Zellzahl wurde nach 3, 5, 7, 10 und 12 Tagen mikroskopisch bestimmt.

Die Ergebnisse sind nachfolgend in Tabelle 2 wiedergegeben.

Tabelle 2

| Zellinie | Konz. (µM/ml) 7-OH-1,2-BP | Zellzahl (x$10^4$) nach den Tagen | | | | | % Wachstumshemmung gegenüber Kontrolle nach 12 Tagen |
|---|---|---|---|---|---|---|---|
| | | 3 | 5 | 7 | 10 | 12 | |
| U 178 MG Kontrolle | 25 | 15<br>23 | 13<br>57 | 26<br>84 | 27<br>91 | 37<br>141 | 74% |
| TP 41O N Kontrolle | 25 | 8<br>25 | 20<br>45 | 36<br>71 | 42<br>97 | 50<br>132 | 62% |
| TP 242 MG Kontrolle | 25 | 4<br>10 | 6<br>15 | 8<br>23 | 10<br>49 | 12<br>57 | 79% |
| TP 336 MG Kontrolle | 10 | 7<br>13 | 11<br>25 | 22<br>39 | 18<br>34 | 18<br>45 | 60% |
| A 431 Kontrolle | 10 | 4<br>6 | 10<br>24 | 3<br>45 | 12<br>56 | 21<br>87 | 76% |

Die Tabelle zeigt, daß bei allen untersuchten Zellinien eine deutliche Hemmung eintrat, wobei bei TP 242 MG mit 25 µM/ml eine nahezu 80%-ige Hemmung der Proliferation gegenüber der Kontrolle beobachtet wurde. Von besonderer Bedeutung ist die Wachstumshemmung der Gehirntumorzellen U 178 MG, TP 410 N, TP 242 MG und TP 336 MG, die - abgesehen von den Interferonen - bisher mit keiner zur Verfügung stehenden Substanz erreicht werden konnte.

**Vergleichsversuch:**

Die Erstellung der Wachstumskurve wurde wiederholt mit dem Unterschied, daß anstelle von 7-OH-1,2-BP Cumarin in den entsprechenden Konzentrationen eingesetzt wurde.

Sowohl im Vorversuch als auch in den Ansätzen zur Erstellung einer Wachstumskurve konnte kein signifikanter Unterschied der mit Cumarin behandelten Zellproben gegenüber der Kontrolle beobachtet werden.

Auf die untersuchten Zellinien hat demgemäß nur 7-Hydroxy-1,2-benzopyron, nicht aber die in 7-Stellung unsubstituierte Verbindung wachstumshemmende Wirkung.

**Beispiel 2**

**Wachstumshemmende Wirkung von 7-OH-1,2-BP auf Mamma-Karzinom-Zellen**

Die Zellinie MCF 7 aus Mamma-Karzinom wurde in DMEM-S 10, angereichert mit 10%-igem FCS 10 Tage lang auf übliche Weise kultiviert. Die Zellen wurden anschließend mit den in Figur 1 angegebenen Konzentrationen von 7-OH-1,2-BP inkubiert und anschließend die Überlebendsrate und Zellzahl mit dem Ethidiumbromid/Acridin orange Verfahren auf bekannte Weise photometrisch bestimmt. Die Ergebnisse sind in Figur 1 wiedergegeben.

Sie zeigen deutlich die cytostatische Wirkung von 7-OH-1,2-BP auf Mamma-Karzinomzellen.

**Beispiel 3**

**Antineoplastische Wirkung von 7-OH-1,2-BP auf humane anaplastische Astrocytomzellen**

Das Verfahren gemäß Beispiel 2 wurde wiederholt, mit dem Unterschied, daß Zellen der Linie 6-UVW aus humanem anaplastischen Astrocytom eingesetzt und in DMEM:HAM's F 12 Medium (1:1) angereichert mit L-Glutamin und 10% FCS eingesetzt wurden. Die Ergebnisse sind in Figur 2 wiedergegeben.

Sie zeigen, daß 7-OH-1,2-BP cytostatische Wirksamkeit auch auf humane anaplastische Astrocytomzellen ausübt.

**Beispiel 4**

**Wachstumshemmende wirkung von 7-Hydroxy-1,2-Benzopyron und Derivaten desselben auf verschiedene Tumorzellinien**

Die Wirkung von 7-Hydroxy-1,2-Benzopyron (7-OH-Cumarin), 6,7-Hydroxy-1,2-Benzopyron (Esculetin), 6-$\beta$-D-Glucopyranosyloxy-7-Hydroxy-1,2-Benzopyron (Esculin) und 6-Methoxy-7-Hydroxy-1,2-Benzopyron (Scopoletin) wurde an den folgenden Zellinien untersucht: Die Ergebnisse für Esculetin, Esculin und Scopoletin dienen nur zu Illustrationszwecken. Es handelt sich nicht um Ausführungsarten der Erfindung.

| | |
|---|---|
| Glioblastom | Tp242MG<br>Tp483MG |
| Neuroblastom | Tp410N |
| Leukämische | K562<br>Daudi |
| Hypernephrom | CAK I-1<br>CAK I-2 |
| Blasentumor | HCV |
| Brustkrebs | MCF-7<br>Z-R-75-1 |
| Prostatakarzinom | GV 1<br>AtT-20 |
| Melanom | A-375<br>G-361 |

Die Zellinien wurden wie in Beispiel 1 angegeben kultiviert.

Die antiproliferative Wirkung der erfindungsgemäßen 1,2-Benzopyron-Derivate auf die jeweiligen Tumorzellinien wurde mit Hilfe des $^{3}$H-Thymidineinbaus sowie anhand von Wachstumskurven nachgewiesen.

**$^{3}$H-Thymidin-Assay**

In einer 24-Lochplatte wurden jeweils $10^4$ Zellen der zu untersuchenden Linie je Loch ausgesät. Die Zellen waren etwa 48 Stunden später halbkonfluent; daraufhin wurde das Medium gewechselt und durch ein den jeweiligen Wirkstoff enthaltendes Medium ersetzt (vgl. Tabelle 3). Nach weiteren 48 Stunden wurden jedem Loch 0,25 $\mu$ Ci $^{3}$H-Thymidin (Ammersham) zugesetzt und die Zellen wurden nochmals 24 Stunden lang inkubiert. Anschließend wurden die Kulturen zweimal mit eiskaltem PBS gewaschen und die hochmolekulare $^{3}$H-Radioaktivität mit 5%-iger Trichloressigsäure 1 Stunde lang bei 4 °C gefällt. Nach dem Waschen der Zellen in PBS wurde die $^{3}$H-Radioaktivität mit 0,3 M NaOH solubilisiert und im Counter bestimmt. Die Wachstumshemmung wurde durch Vergleich des Thymidineinbaus der wirkstoffbehandelten Zellen mit unbehandelten Zellen ermittelt. Die Ergebnisse sind nachfolgend in Tabelle 3 wiedergegeben.

**Tabelle 3**

| Wirkstoffkonzentration (µM/ml) | $^3$H-Thymidineinbau (%) von Kontrolle 7-OH-1,2-BP | Esculetin | Esculin | Scopoletin |
|---|---|---|---|---|
| **A) Glioblastomzellen Tp242MG/Tp483MG** | | | | |
| 1 | 93/97 | 96/98 | 100/99 | 99/100 |
| 2 | 91/95 | 96/94 | 98/97 | 93/95 |
| 4 | 91/89 | 89/85 | 95/89 | 85/89 |
| 8 | 84/81 | 87/77 | 81/83 | 87/86 |
| 16 | 73/69 | 75/73 | 73/74 | 84/78 |
| 25 | 55/56 | 72/69 | 68/66 | 79/72 |
| 50 | 43/39 | 60/62 | 65/54 | 74/67 |
| 100 | 39/32 | 44/55 | 61/51 | 61/63 |
| 250 | 35/27 | 32/39 | 53/48 | 52/54 |
| 500 | 32/25 | 21/28 | 49/45 | 48/50 |
| **B) Neuroblastom-Zellen TP 410 N** | | | | |
| 1 | 96 | 94 | 100 | 100 |
| 2 | 91 | 86 | 96 | 98 |
| 4 | 80 | 75 | 85 | 95 |
| 8 | 73 | 69 | 76 | 87 |
| 16 | 67 | 64 | 69 | 82 |
| 25 | 50 | 48 | 62 | 74 |
| 50 | 48 | 42 | 54 | 71 |
| 100 | 42 | 40 | 43 | 65 |
| 250 | 36 | 34 | 38 | 62 |
| 500 | 21 | 29 | 30 | 54 |

**Tabelle 3 (Fortsetzung)**

| Wirkstoffkonzentration (µM/ml) | $^{3}$H-Thymidineinbau (%) von Kontrolle 7-OH-1,2-BP | Esculetin | Esculin | Scopoletin |
|---|---|---|---|---|
| **C) Leukämische Zellen Daudi/K-562** | | | | |
| 1 | 98/100 | 94/97 | 100/100 | 96/100 |
| 2 | 89/91 | 96/95 | 98/100 | 94/97 |
| 4 | 82/84 | 83/84 | 93/96 | 80/90 |
| 8 | 76/76 | 75/77 | 87/88 | 79/85 |
| 16 | 63/67 | 69/62 | 80/81 | 71/82 |
| 25 | 54/53 | 63/58 | 71/72 | 64/71 |
| 50 | 49/47 | 61/50 | 69/70 | 58/66 |
| 100 | 38/42 | 57/47 | 52/58 | 53/52 |
| 250 | 35/39 | 54/44 | 48/51 | 49/47 |
| 500 | 28/37 | 39/31 | 44/40 | 41/44 |
| **D) CAKI-Zellen CAKI-1/CAKI-2** | | | | |
| 1 | 100/95 | 96/98 | 100/99 | 100/99 |
| 2 | 98/92 | 93/94 | 94/96 | 98/96 |
| 4 | 90/88 | 88/90 | 92/90 | 87/84 |
| 8 | 81/77 | 80/87 | 83/84 | 81/80 |
| 16 | 76/71 | 62/82 | 78/73 | 80/76 |
| 25 | 62/65 | 58/69 | 74/72 | 73/71 |
| 50 | 57/54 | 53/65 | 65/66 | 69/66 |
| 100 | 49/46 | 45/54 | 60/50 | 67/61 |
| 150 | 44/39 | 40/53 | 53/56 | 62/59 |
| 500 | 40/35 | 32/50 | 47/52 | 55/53 |

**Tabelle 3 (Fortsetzung)**

| Wirkstoffkonzentration (µM/ml) | $^3$H-Thymidineinbau (%) von Kontrolle 7-OH-1,2-BP | Esculetin | Esculin | Scopoletin |
|---|---|---|---|---|
| **E) HCV-Zellen** | | | | |
| 1 | 98 | 99 | 100 | 97 |
| 2 | 95 | 94 | 98 | 90 |
| 4 | 88 | 87 | 95 | 86 |
| 8 | 82 | 83 | 89 | 81 |
| 16 | 74 | 78 | 86 | 77 |
| 25 | 69 | 76 | 83 | 69 |
| 50 | 63 | 71 | 79 | 65 |
| 250 | 57 | 64 | 76 | 60 |
| 500 | 50 | 62 | 75 | 57 |
| **F) Brustkrebszellen MC7/Z-R-75-1** | | | | |
| 1 | 98/96 | 100/98 | 99/100 | 100/98 |
| 2 | 94/91 | 98/96 | 91/97 | 97/96 |
| 4 | 88/82 | 92/89 | 88/89 | 94/91 |
| 8 | 82/73 | 85/84 | 80/85 | 86/83 |
| 16 | 75/65 | 76/77 | 71/75 | 81/79 |
| 25 | 69/55 | 69/70 | 69/72 | 76/72 |
| 50 | 62/49 | 64/61 | 62/64 | 73/64 |
| 100 | 51/43 | 58/53 | 51/56 | 69/57 |
| 250 | 43/39 | 50/46 | 49/51 | 62/55 |
| 500 | 29/27 | 42/39 | 42/47 | 54/50 |

**Tabelle 3 (Fortsetzung)**

| Wirkstoffkonzentration (µM/ml) | $^3$H-Thymidineinbau (%) von Kontrolle 7-OH-1,2-BP | Esculetin | Esculin | Scopoletin |
|---|---|---|---|---|
| **G) Prostatakarzinomzellen GV-1/At T 20** | | | | |
| 1 | 100/98 | 97/93 | 97/100 | 100/98 |
| 2 | 89/89 | 93/89 | 95/94 | 98/94 |
| 4 | 83/85 | 88/86 | 85/89 | 91/89 |
| 8 | 75/78 | 84/75 | 84/83 | 88/84 |
| 16 | 69/65 | 78/75 | 79/72 | 82/76 |
| 25 | 63/60 | 75/67 | 76/69 | 78/68 |
| 50 | 51/62 | 63/62 | 68/64 | 71/62 |
| 100 | 39/44 | 60/55 | 60/58 | 63/57 |
| 250 | 36/38 | 58/49 | 51/49 | 61/49 |
| 500 | 27/31 | 50/44 | 43/44 | 51/43 |
| **H) Melanomzellen A-375/G-361** | | | | |
| 1 | 100/94 | 98/97 | 100/98 | 99/100 |
| 2 | 92/91 | 91/93 | 96/94 | 96/98 |
| 4 | 86/77 | 89/90 | 93/90 | 90/96 |
| 8 | 81/77 | 84/88 | 87/86 | 89/88 |
| 16 | 72/74 | 75/72 | 82/81 | |
| 25 | 64/68 | 70/68 | 78/74 | 87/85 |
| 50 | 53/58 | 65/60 | 71/70 | 78/85 |
| 100 | 49/53 | 63/58 | 64/63 | 75/76 |
| 250 | 47/42 | 59/52 | 60/60 | 75/71 |
| 500 | 44/38 | 55/49 | 57/54 | 70/67 |

**Erstellen von Wachstumskurven**

Die Wachstumskurven der jeweiligen Zellinien wurden in Anwesenheit der unten angegebenen Wirkstoffe bestimmt und mit Kontrollen verglichen, die ohne Wirkstoff kultiviert worden waren.

Die jeweilige Wirkstoffkonzentration wurde so gewählt, daß der $^3$H-Thymidineinbau zu 50% inhibiert war. Die unter diesem Gesichtspunkt für die jeweiligen Wirkstoffe und Zellinien eingesetzten Wirkstoffkonzentration in µM/ml sind nachfolgend in Tabelle 4 angegeben.

Tabelle 4

| Zellinien | 7-OH-C | Esculetin | Esculin | Scopoletin |
|---|---|---|---|---|
| Tp 242 MG | 25 | 75 | 500 | 500 |
| Tp 483 MG | 25 | 150 | 100 | 500 |
| Tp 410 N | 25 | 25 | 75 | 500 |
| K 562 | 50 | 50 | 250 | 175 |
| Daudi | 50 | 250 | 100 | 250 |
| CAKI-1 | 100 | 50 | 375 | 500 * |
| CAKI-2 | 75 | 500 | 500 * | 500 * |
| HCV | 500 | 500 * | 500 * | 500 * |
| MCF-7 | 100 | 250 | 100 * | 500 * |
| Z-R-75-1 | 50 | 175 | 250 | 500 * |
| GV-I | 75 | 500 | 250 | 500 |
| AtT 20 | 50 | 250 | 250 | 250 |
| A-375 | 100 | 500 * | 500 * | 500 * |
| G-361 | 125 | 500 | 500 * | 500 * |

* Maximale Wirkstoff-Dosis, die eingesetzt werden kann, jedoch nicht zu einer 50%-igen Wachstumsinhibition führt.

Zur Erstellung der Wachstumskurven wurden jeweils $10^4$ Zellen je Platte ausgesät und in Gegenwart des jeweiligen Wirkstoffes bzw. als Kontrolle inkubiert. Nach 2, 5, 7 und 10 Tagen wurde die Zellzahl mikroskopisch bestimmt. Die Ergebnisse sind in den Figuren 12 bis 24 wiedergegeben.

Die dort angegebenen Werte repräsentieren die durchschnittliche Zellzahl von jeweils drei Parallelansätzen.

**Beispiel 5**

**Hemmung des Wachstums von Blasentumor- und erythroleukämischen Zellen durch 7-OH-1,2-BP**

Zellen aus 3 verschiedenen Blasentumoren sowie erythroleukämische Zellen (K 562) wurden in 3 Parallelansätzen 20 Stunden lang mit steigenden Konzentrationen von 7-OH-1,2-BP (vgl. Figur 3) jeweils in 96-Loch-Platten inkubiert, gewaschen und mit Medium versetzt. Anschließend wurde die Zellzahl photometrisch nach Alley M.C. et al., Cancer Research 48, 589-601 (1988) bestimmt. Bei dem Verfahren wird den mit Medium versetzten Zellen ein Farbstoff zugegeben, der von Zellen mit mitochondrialer Aktivität so umgewandelt wird, daß eine photometrische Bestimmung der Zellzahl möglich ist.

Die Ergebnisse sind in Figur 3 wiedergegeben.

Sie zeigen, daß es bei den untersuchten Zellinien dosisabhängig zu einer Hemmung der mitochondrialen Aktivität kommt, die kausal auf Zellwachstumshemmung zurückzuführen ist.

**Beispiel 6**

**Synergistische Wirkung von 7-OH-1,2-BP in Kombination mit 5-Fluorouracil (5-FU) und mit cis-Platin**

Es wurden Zellen aus einem Colon-Adenokarzinom und aus einem bronchoalveolären Karzinom verwendet. Die Zellen wurden kultiviert und nach dem Verfahren von Steel, Int. J. Radiat. Oncol. Biol. Phys. 5, 85-91 (1979) die synergistische Wirksamkeit bei der Zellwachstumshemmung von 7-OH-1,2-BP jeweils in Kombination mit 5-FU und mit cis-Platin untersucht.

Zu diesem Zweck wurden zunächst einzelne Dosis-Responskurven jeweils für 5-FU, cis-Platin und 7-OH-1,2-BP bezüglich der Zellwachstumshemmung aufgenommen. Anschließend wurden für jede der Kombinationen von beiden Kurven die Punkte gleicher biologischer Aktivität als Referenzpunkte für jene Dosismengen verwendet, die in einem Kombinationsversuch (5-FU + 7-OH-1,2-BP oder cis-Platin + 7-OH-1,2-BP) verwendet werden sollten. Mit diesen Konzentrationen wurden wiederum Dosisresponskurven aufgenommen.

Die Ergebnisse sind in Figur 4a) und 4b) wiedergegeben.

Sie zeigen, daß beide Kombinationen eine synergistische Wirksamkeit bei der Hemmung des Zellwachstums der untersuchten Zellinien aufwiesen.

**Beispiel 7**

**Synergistische Wirkung von 7-OH-1,2-BP in Kombination mit Tumor-Nekrose-Faktor (TNF)**

Es wurden verschiedene Zellinien (DU-145, PC-3 und LNCaP) aus Prostatakarzinomen verwendet.

Das Zellwachstum wurde wie in Beispiel 1 angegeben in einer Lochplatte bestimmt, wobei die Zellen mit 7-OH-1,2-BP allein, TNF allein, der Kombination aus 7-OH-1,2-BP und TNF sowie ohne Wirkstoffzusatz (Kontrolle) kultiviert wurden.

Die Ergebnisse sind nachfolgend in Tabelle 5 angegeben.

Tabelle 5

| Zellzahl x $10^4$ | | | | |
|---|---|---|---|---|
| Zellinie | Kontrolle | 7-OH-1,2-BP (500 µM) | TNF [1 nM] | 7-OH-1,2-BP/TNF (500 µM/1nM) |
| DU-145 | 51,86±3,06 | 38,30±2,92 | 55,39±0,68 | 29,97±3,69 |
| PC-3 | 18,58±1,05 | 11,51±0,63 | 18,39±0,74 | 6,97±0,13 |
| LNCaP | 61,40±3,70 | 11,0±1,10 | 15,60±2,50 | 1,60±0,99 |
| Ausgangszellzahl: 3 x $10^4$ Zellen/Loch; Einwirkungsdauer: DU-145, PC-3: 4 Tage; LNCaP:6 Tage | | | | |

Die Ergebnisse zeigen, daß die Kombination von 7-OH-1,2-BP mit TNF zu einem überadditiven Hemmeffekt des Zellwachstums führt.

**Beispiel 8**

**Steigerung der Wachstumshemmung durch Kombination von 7-OH-1,2-BP mit Testosteron**

LNCaP-Zellen wurden wie in den vorangehenden Beispielen angegeben in einer Lochplatte kultiviert, mit dem Unterschied, daß das Medium zuvor mit Holzkohle von gegebenenfalls vorhandenen androgenen Substanzen befreit worden war.

Das Zellwachstum wurde in Gegenwart von 250 bzw. 500 µM 7-OH-1,2-BP/ml Medium kombiniert mit 20 nM Testosteron je Loch bestimmt. Die Ergebnisse sind in Figur 9 wiedergegeben. Sie zeigen, daß in Gegenwart von 500 µM 7-OH-1,2-BP eine nahezu vollständige Hemmung des Zellwachstums erreicht wird. Ferner weist der Vergleich mit der Verbindung 3-OH-1,2-BP nach, daß letztere im wesentlichen unwirksam ist.

**Beispiel 9**

**Toxizität von 7-OH-1,2-BP beim Menschen**

60 Patienten, die an unterschiedlichen, malignen Tumoren litten, wurden in einer Toleranzstudie mit 7-OH-1,2-BP behandelt. Die Anfangsdosis betrug 50 mg der Substanz/Tag und die Dosis wurde kontinuierlich bis zu 2000 mg/Tag erhöht. Die Patienten wurden während 8 aufeinanderfolgender Wochen einmal wöchentlich auf systemische Toxizitätszeichen und organische Funktionsstörungen hin untersucht. Da am Ende der achten Woche keinerlei Nebenwirkungen aufgetreten waren, wurde die Studie unter Durchführung der üblichen laborchemischen Kontrollen fortgesetzt und die Tagesdosis schrittweise bis auf 7000 mg/Tag erhöht. Toxische Nebenwirkungen konnten selbst bei Langzeitverabreichung einer Dosismenge von 7000 mg/Tag nicht beobachtet werden.

**Beispiel 10**

**Immunmodulierende Wirkung von 7-OH-1,2-BP**

Der Einfluß von 7-OH-1,2-BP auf die Lymphokinfreisetzung durch humane mononucleare Zellen (MNC) wurde untersucht, indem man die Zellen mit verschiedenen Konzentrationen (vgl. Figuren 5 und 6) von 7-OH-1,2-BP gelöst in Methanol 48 Stunden lang inkubierte. Die Stammlösung enthielt 10 mg 7-OH-1,2-BP/ml Methanol und wurde mit RPMI 1640 auf die jeweils gewünschte Konzentration eingestellt. Die Zellen wurden aus dem peripheren Blut gesunder Spender durch Dichtegradienten-Zentrifugation nach dem von Bøyum in Scand. J. Clin. Lab. Invest. **21**, 77 (1968) beschriebenen Verfahren isoliert und nach 3-maligem Waschen mit physiologischer Kochsalzlösung in RPMI mit einem Gehalt an 100 U/ml Penicillin, 100 µg/ml Streptomycin und 2 mM Glutamin (Vollmedium) suspendiert. Die Lymphokine Interleukin-1 (IL 1) und Interleukin-6 (IL 6) wurden auf bekannte Weise in vivo nach Induktion mit Phythämagglutinin (PHA), Concanavalin A (ConA), Lipopolysaccharid (LPS) bzw. OKT 3 und Zusatz steigender Dosismengen von 7-OH-1,2-BP bestimmt. Die Messung erfolgte in einem Elisa an Mikrotiterplatten als Festphase (fixierte monoklonale Antikörper gegen das jeweilige Lymphokin, polyklonale Kaninchen-Antikörper gegen das gebundene Lymphokin, Kaninchen-Anti-IgG gekoppelt an alkalische Phosphatase als Marker und p-Nitrophenylphosphat als Substrat).

Die Ergebnisse sind in den Figuren 5 und 6 wiedergegeben.

Sie zeigen, daß IL 1 und IL 6 nach Stimulieren mit 7-OH-1,2-BP im Dosisbereich von 3,6 bis 33 µg/ml vermehrt freigesetzt wurden. (Die Hemmung im höheren Dosisbereich beruht auf dem cytotoxischen Effekt des als Lösungsmittel verwendeten Methanols). Der Wirkstoff regt demgemäß in niedrigen Konzentrationen die Freisetzung von Immunmodulatoren an und wirkt dementsprechend direkt immunmodulatorisch.

Ferner wurde die Wechselwirkung verschiedener Cytokine untereinander nach Stimulieren von MNC mit 7-OH-1,2-BP (10 µg/ml) und LPS (10 bis 100 pg/ml) an den MNC von 19 Spendern untersucht. In 6 aus 19 Fällen (32%) konnte eine Costimulation von TNF-alpha, Il-1 und Il-6 nachgewiesen werden. Die Berechnung des Spearman Korrelationskoeffizienten zeigte eine signifikante Korrelation zwischen jedem der 3 Cytokine (Il-1-$\beta$:Il-6 $p = 0{,}022$, Il-1-$\beta$:TNF-$\alpha$ $p = 0{,}007$, Il-6:TNF-$\alpha$ $p = 0{,}007$).

Die Ergebnisse sind in Figur 7 graphisch dargestellt.

**Beispiel 11**

**Chemopräventive Eigenschaften von 7-OH-1,2-BP**

Die chemopräventiven Eigenschaften von 7-OH-1,2-BP wurden an transgenen Mäusen des Typs Onco Maus™ (Mammadrüsen-Neoplasmen) überprüft:
Es wurde untersucht, inwieweit 7-OH-1,2-BP in der Lage ist, die Tumorentstehung zu verhindern oder zu verzögern und die Überlebenszeit der Tiere zu verlängern. 7-OH-1,2-BP wurde in Konzentrationen von 200 µM/ml im Trinkwasser verfüttert.

Die Ergebnisse sind nachfolgend in Tabelle 6 wiedergegeben.

Tabelle 6

| Chemoprävention von 7-OH-1,2-BP gezeigt an transgenen Mäusen | | | |
|---|---|---|---|
| Anzahl der Mäuse ohne 7-OH-Coumarin | Anzahl der Mäuse mit 7-OH-1,2-BP | Tumorinzidenz | % |
| 28 | - | 9/28 | 32 |
| - | 38 | 3/38 | 8 |

**Beispiel 12**

**Wachstumshemmung von "c-myc" exprimierenden Tumorzellen durch 7-OH-1,2-BP**

a) DUKX-Zellen (Ovaryalkarzinom-Zellen von chinesischen Hamstern), die "c-myc" Produkte überexprimieren, wurden nach Standardbedingungen kultiviert, wobei zwei Vergleichsgruppen angezogen wurden.

Bei einer Gruppe enthielt das Medium 100 µg/ml 7-OH-1,2-BP, während die zweite Gruppe als Kontrollgruppe ohne den Wirkstoff kultiviert wurde.

Die Entwicklung der Kulturen wurde nach 24, 48 und 72 Stunden photographisch festgehalten; die Ergebnisse sind in Figur 10 wiedergegeben. Sie zeigen die dramatische Hemmung des Zellwachstums in Gegenwart von 7-OH-1,2-BP (Figur 10, $b_1$ bis $b_3$).

b) Die Untersuchungen gemäß a) wurden wiederholt, mit dem Unterschied, daß die in Gegenwart von 7-OH-1,2-BP kultivierten Zellen nach 24 Stunden von dem Wirkstoff befreit, mit Trypsin behandelt, frisch ausgesät und das Zellwachstum nach weiteren 48 Stunden in 7-OH-1,2-BP freiem Medium photographisch festgehalten wurde.

Zum Vergleich wurden entsprechende Kulturen 24 Stunden lang mit 7-OH-1,2-BP kultiviert, mit Trypsin behandelt, und frisch in einem 7-OH-1,2-BP enthaltenden Medium ausgesät.

Das Zellwachstum wurde ebenfalls nach 48 Stunden photographisch festgehalten.

Die Ergebnisse sind in Figur 11 wiedergegeben.

Sie zeigen ein beträchtliches Zellwachstum derjenigen Kulturen, bei denen das 7-OH-1,2-BP nach 24 Stunden entfernt und während der nachfolgenden Kultivierungsperiode nicht ersetzt worden war (vgl. Figur 11$b_1$). Demgegenüber ist das Zellwachstum derjenigen Kulturen, bei denen auch die zweite Kultivierungsperiode in Gegenwart von 7-OH-1,2-BP stattfand, nahezu vollständig gehemmt (Figur 11$b_2$).

Die Ergebnisse lassen darauf schließen, daß für die Wachstumshemmung von Tumorzellen mit Onkogen-Überexpression die permanente Verfügbarkeit des Wirkstoffes 7-OH-1,2-BP erforderlich ist.

## Patentansprüche

1. Verwendung von 7-OH-1,2-Benzopyron zur Herstellung von Arzneimitteln zur präventiven und/oder zur therapeutischen Behandlung von malignen Tumoren beim Menschen.

2. Verwendung nach Anspruch 1 in Kombination mit üblichen Cytostatika.

3. Verwendung nach Anspruch 1 in Kombination mit Cytokinen.

4. Verwendung nach den Ansprüchen 1 bis 3 in einer Tagesdosis von 50 bis 300 mg zur präventiven Behandlung maligner Tumore beim Menschen.

5. Verwendung nach den Ansprüchen 1 bis 3 in einer Tagesdosis von 300 bis 6000 mg zur therapeutischen Behandlung maligner Tumore beim Menschen.

6. Verwendung nach den Ansprüchen 1 bis 5 zur Herstellung von Arzneimitteln zur Behandlung von Gehirntumoren.

7. Verwendung nach den Ansprüchen 1 bis 5 zur Herstellung von Arzneimitteln zur Behandlung von humanen Nieren-, Prostata-, Haut- oder Lungenkarzinomen oder von Leukämie.

8. Verwendung nach den Ansprüchen 1 bis 5 zur Herstellung von Arzneimitteln zur Behandlung von durch Onkogenexpression induzierten Tumoren.

9. Verwendung nach Anspruch 1 in Kombination mit Hormonen zur Herstellung von Arzneimitteln zur Behandlung hormonabhängiger Tumore.

## Claims

1. Use of 7-OH-1,2-benzopyrone for the production of medicaments for the preventive and/or therapeutic treatment of malignant tumours in humans.

2. Use according to claim 1 in combination with usual cytostatics.

3. Use according to claim 1 in combination with cytokines.

4. Use according to claims 1 to 3 in a daily dosage of 50 to 300 mg for the preventive treatment of malignant tumours in humans.

5. Use according to claims 1 to 3 in a daily dosage of 300 to 6000 mg for the therapeutic treatment of malignant tumours in humans.

6. Use according to claims 1 to 5 for the production of medicaments for the treatment of brain tumours.

7. Use according to claims 1 to 5 for the production of medicaments for the treatment of human renal, prostate, skin or lung carcinomas or of leukaemia.

8. Use according to claims 1 to 5 for the production of medicaments for the treatment of tumours induced by oncogene expression.

9. Use according to claim 1 in combination with hormones for the production of medicaments for the treatment of hormone-dependent tumours.

**Revendications**

1. Utilisation de la 7-OH-1,2-benzopyrone pour la production de médicaments pour le traitement préventif et/ou thérapeutique de tumeurs malignes chez l'homme.

2. Utilisation selon la revendication 1 en association avec la cytostatique traditionnelle.

3. Utilisation selon la revendication 1 en association avec des cytokines.

4. Utilisation selon les revendications 1 à 3 à une dose quotidienne de 50 à 300 mg pour le traitement préventif de tumeurs malignes chez l'homme.

5. Utilisation selon les revendications 1 à 3 à une dose quotidienne de 300 à 600 mg pour le traitement thérapeutique de tumeurs malignes chez l'homme.

6. Utilisation selon les revendications 1 à 5 pour la production de médicaments pour la traitement de tumours cérébrales.

7. Utilisation selon les revendications 1 à 5 pour la production de médicaments pour le traitement de carcinomes humains des reins, de la prostate, de la peau ou des poumons ou bien de la leucémie.

8. Utilisation selon les revendications 1 à 5 pour la production de médicaments pour le traitement de tumeurs induites par l'expression des oncogènes.

9. Utilisation selon la revendication 1 en association avec des hormones pour la production de médicaments pour le traitement de tumeurs hormono-dépendantes.

Fig.1

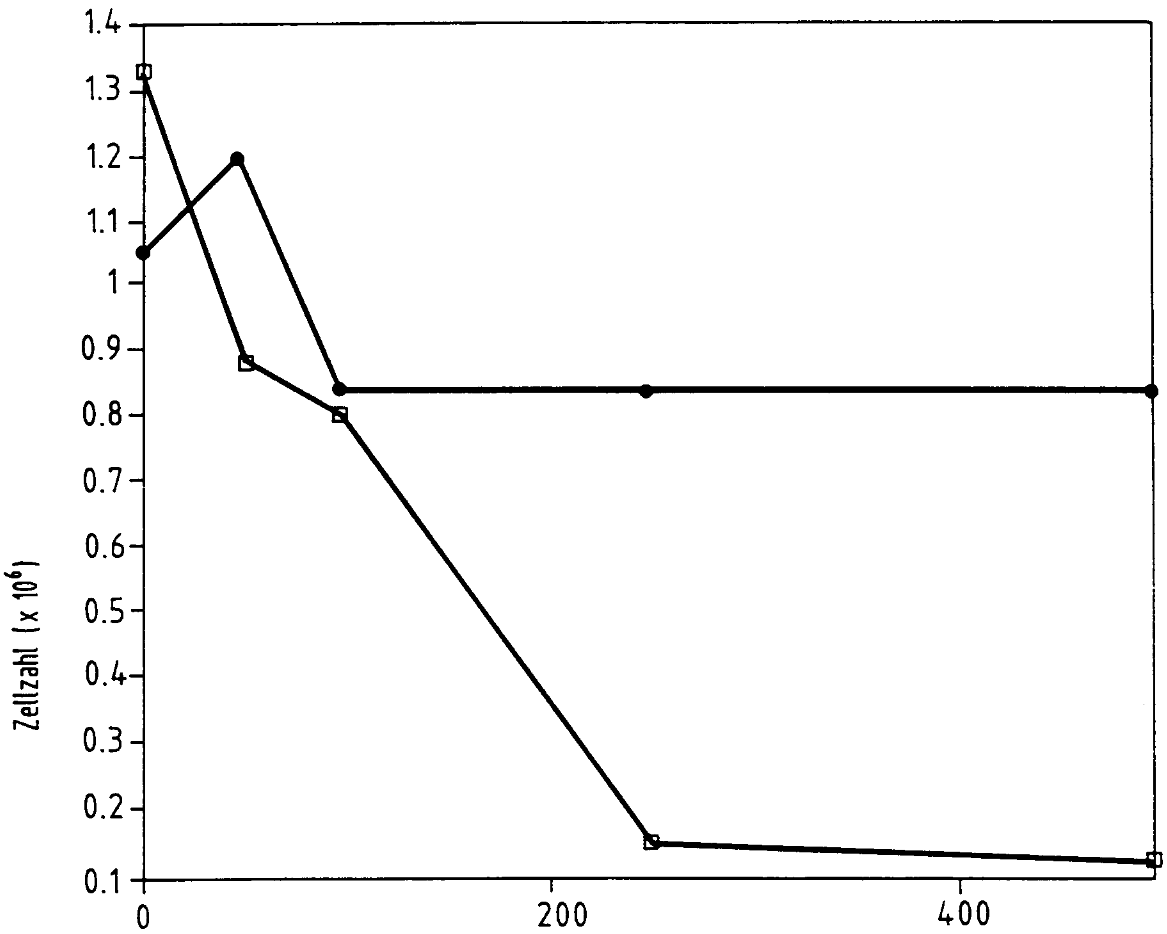


Wirkstoffkonzentration (µM)
Einfluß von 7-Hydroxy-1,2-benzopyron auf MCF 7
□ 7-Hydroxy-1,2-benzopyron
● Kontrolle

Fig. 2

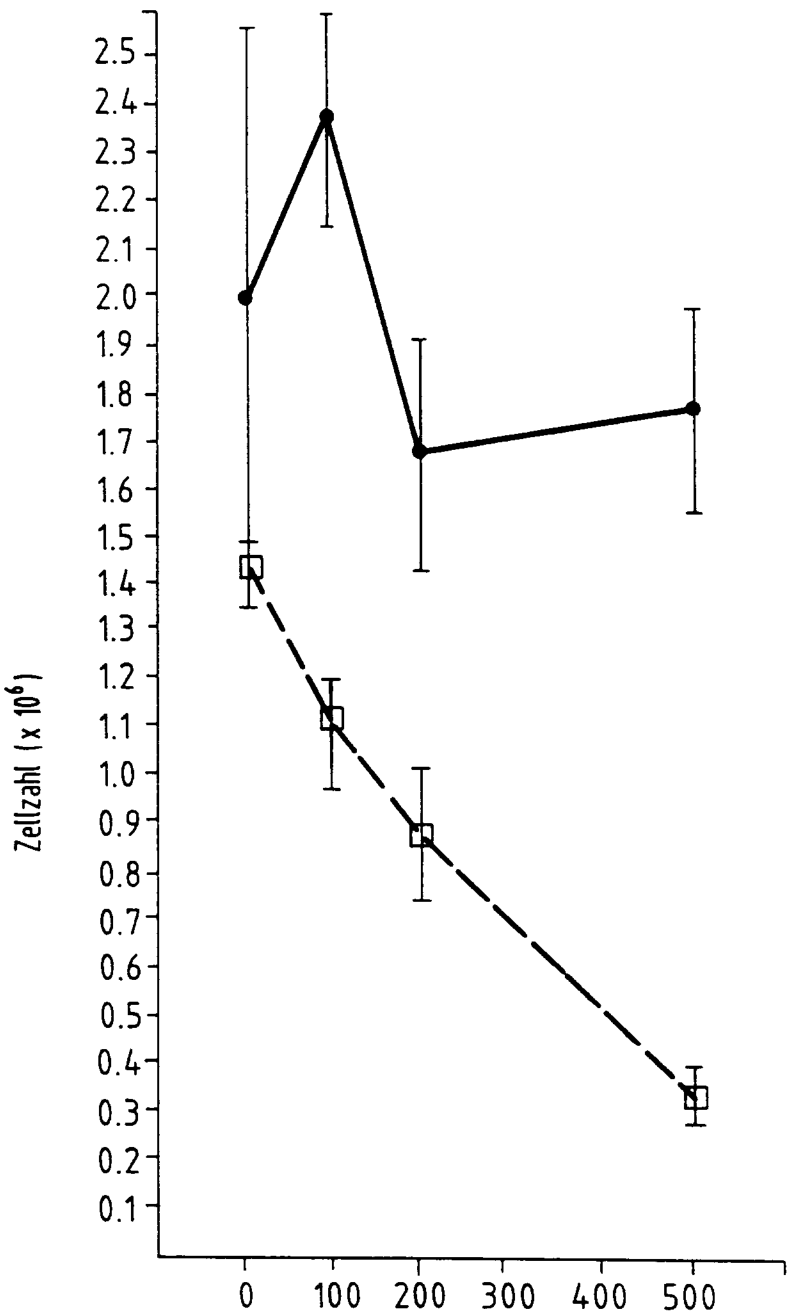


Wirkstoffkonzentration (µM)
Einfluß von 7-Hydroxy-1,2-benzopyron auf 6-UVW

□ 7-Hydroxy-1,2-benzopyron
● Kontrolle

Fig. 3

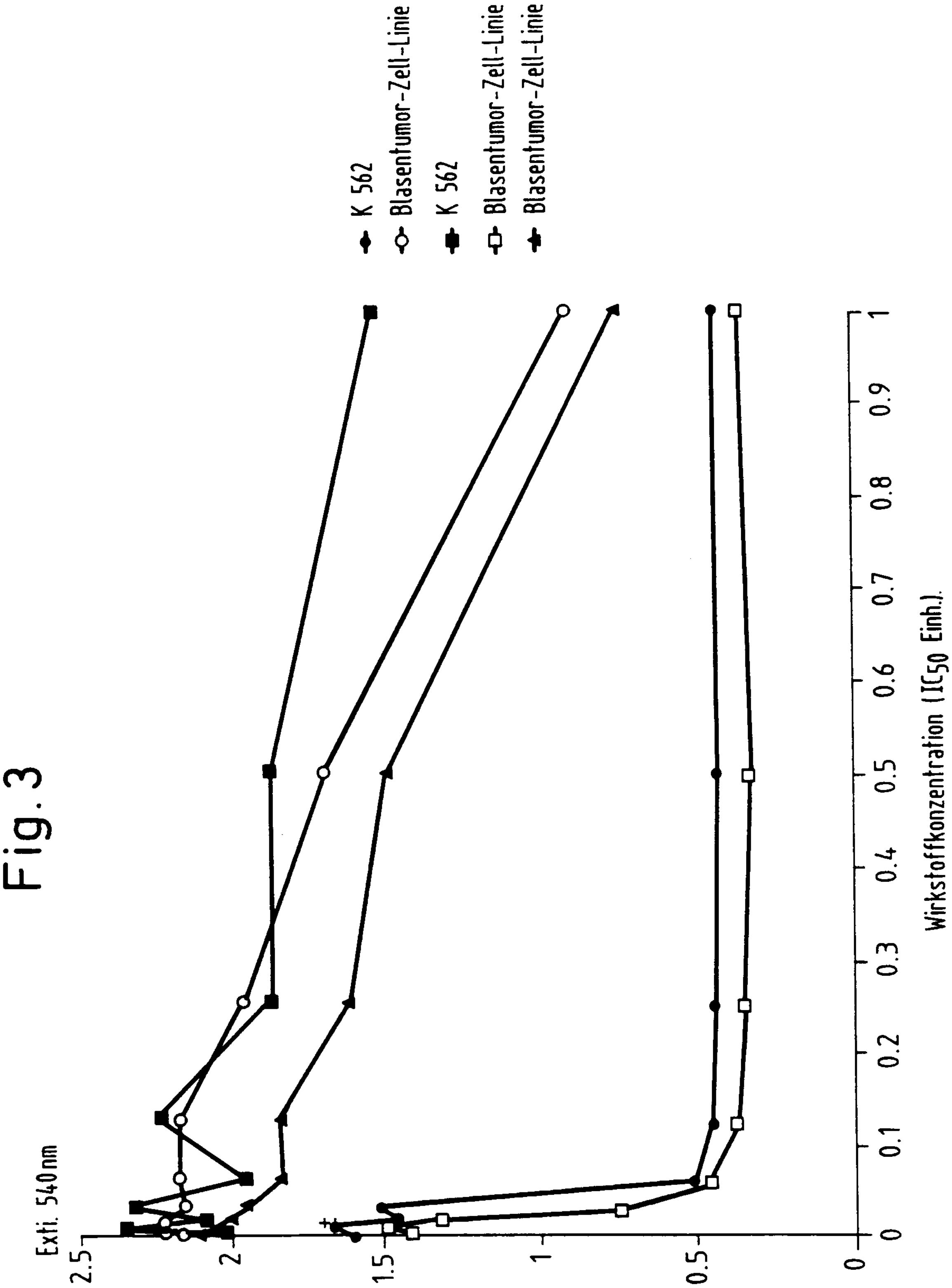
Exti. 540nm
2.5
2
1.5
1
0.5
0
0
0.1
0.2
0.3
0.4
0.5
0.6
0.7
0.8
0.9
1
Wirkstoffkonzentration (IC50 Einh.)
K 562
Blasentumor-Zell-Linie
K 562
Blasentumor-Zell-Linie
Blasentumor-Zell-Linie

Fig.4

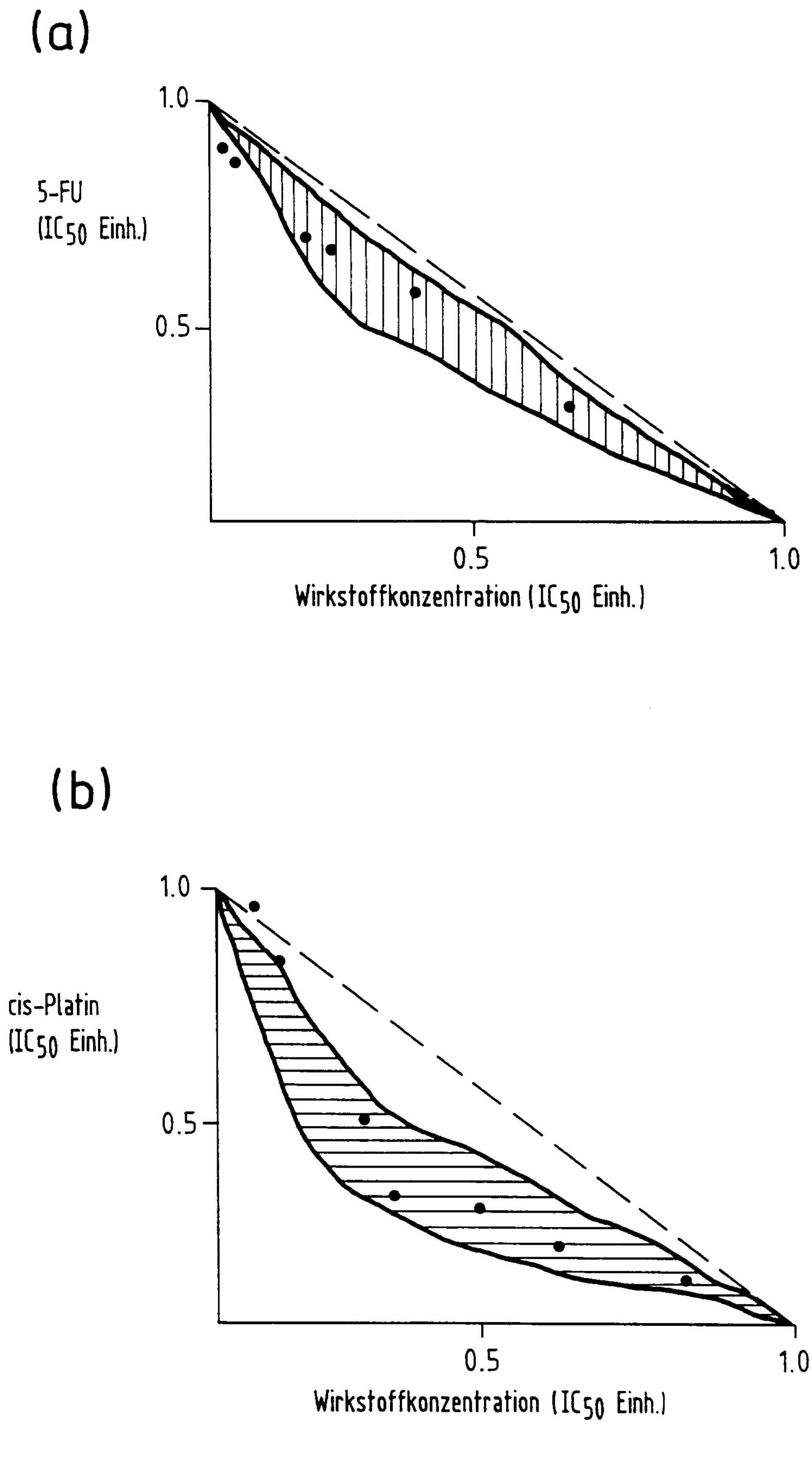
(a)
1.0
5-FU
(IC50 Einh.)
0.5
0.5
1.0
Wirkstoffkonzentration (IC50 Einh.)
(b)
1.0
cis-Platin
(IC50 Einh.)
0.5
0.5
1.0
Wirkstoffkonzentration (IC50 Einh.)

Fig.5

IL 1 - Sekretion nach
Stimulieren mit 7-Hydroxy-1,2-benzopyron

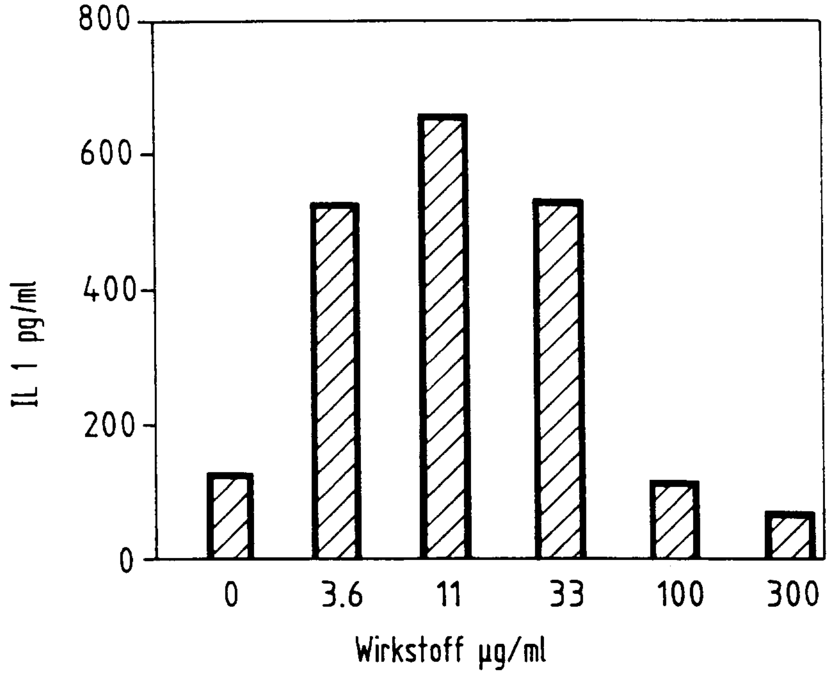


Fig.6

IL 6 - Sekretion nach
Stimulieren mit 7-Hydroxy-1,2-benzopyron

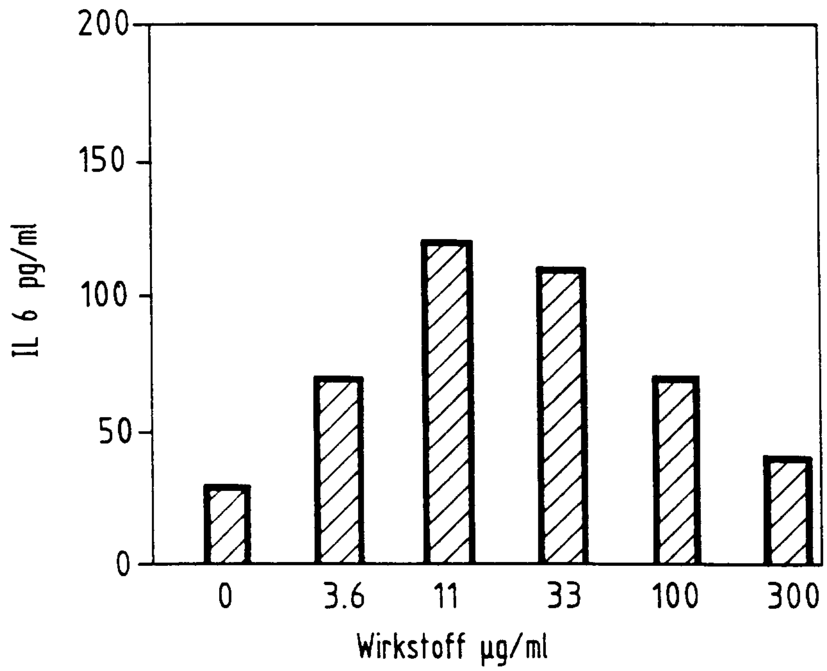

# Fig.7

Costimulation von TNF und IL-1 oder IL-6
durch 7-OH-1,2-BP
in
Kombination mit Endotoxinen
SI = Stimulations-Index

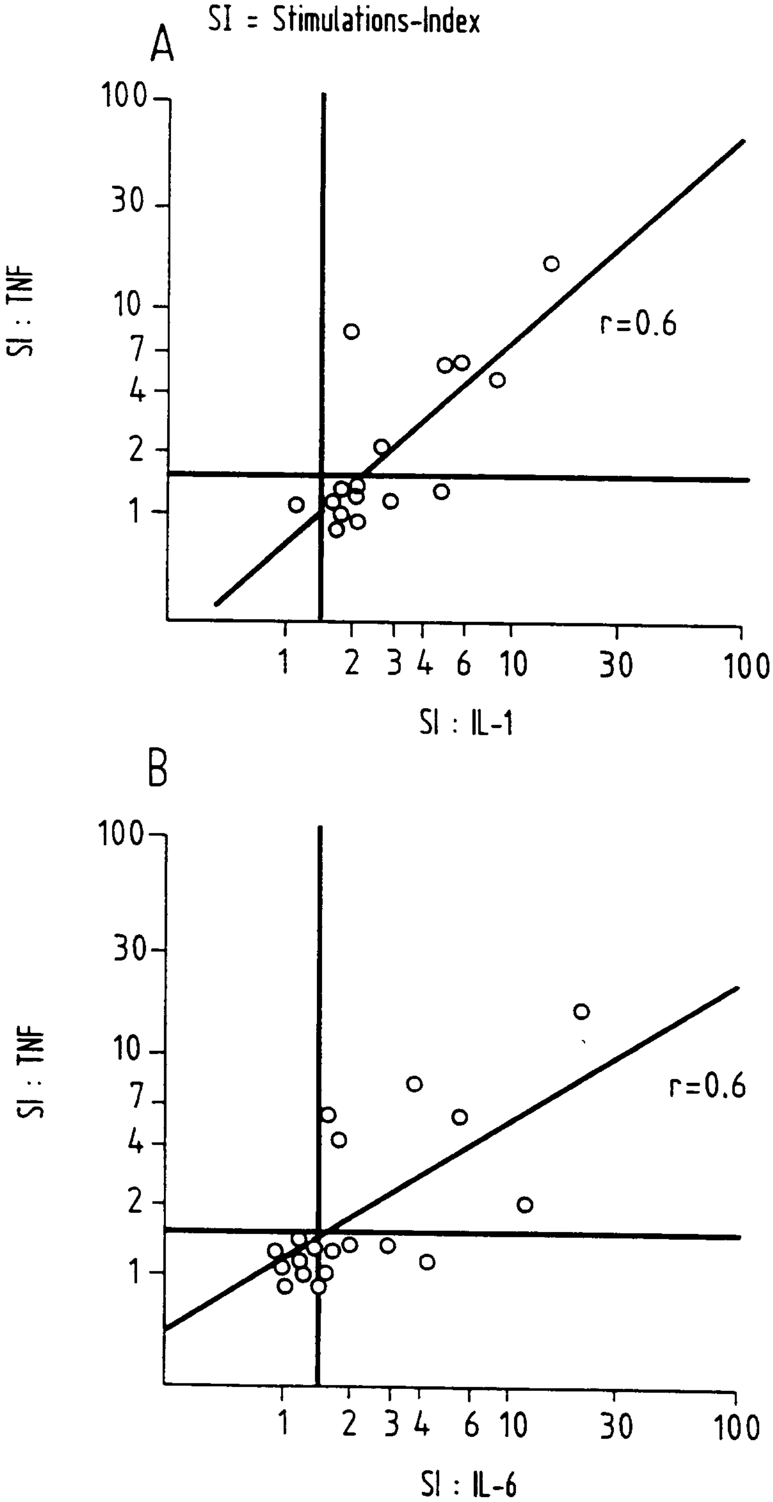

Fig.8

mRNA - Nachweis

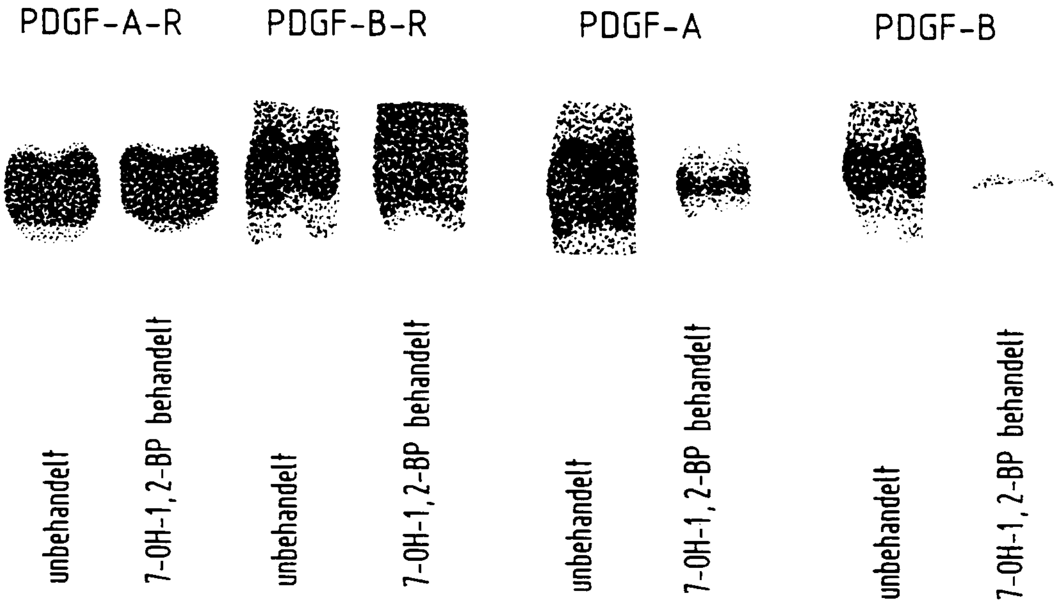

Fig.9

Kombination von 7-OH-1,2-BP mit Testosteron
Hemmwirkung auf LNCaP-Zellen

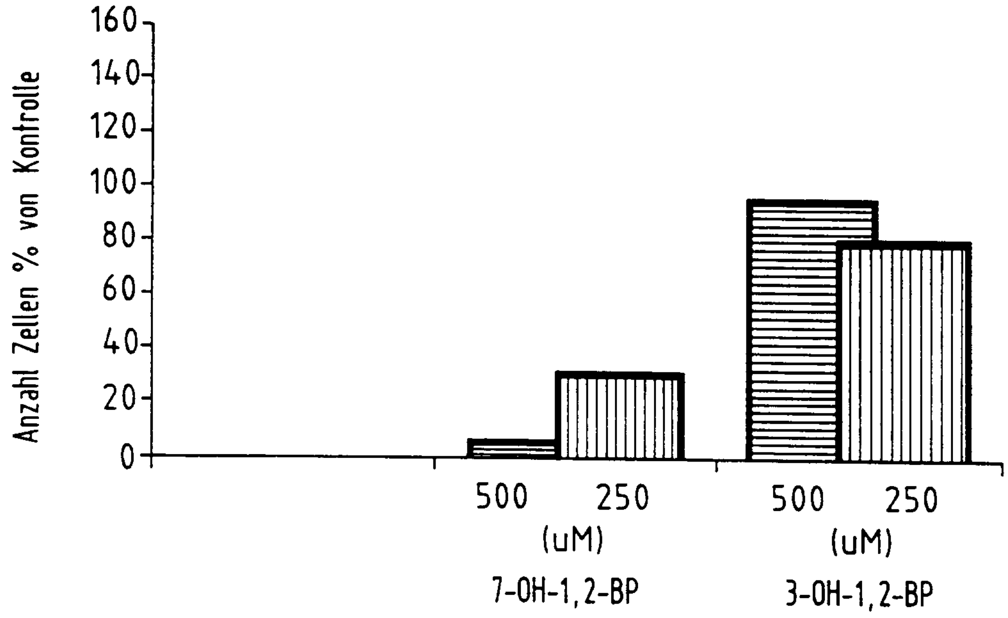

Fig.10

DUKX-Zellen

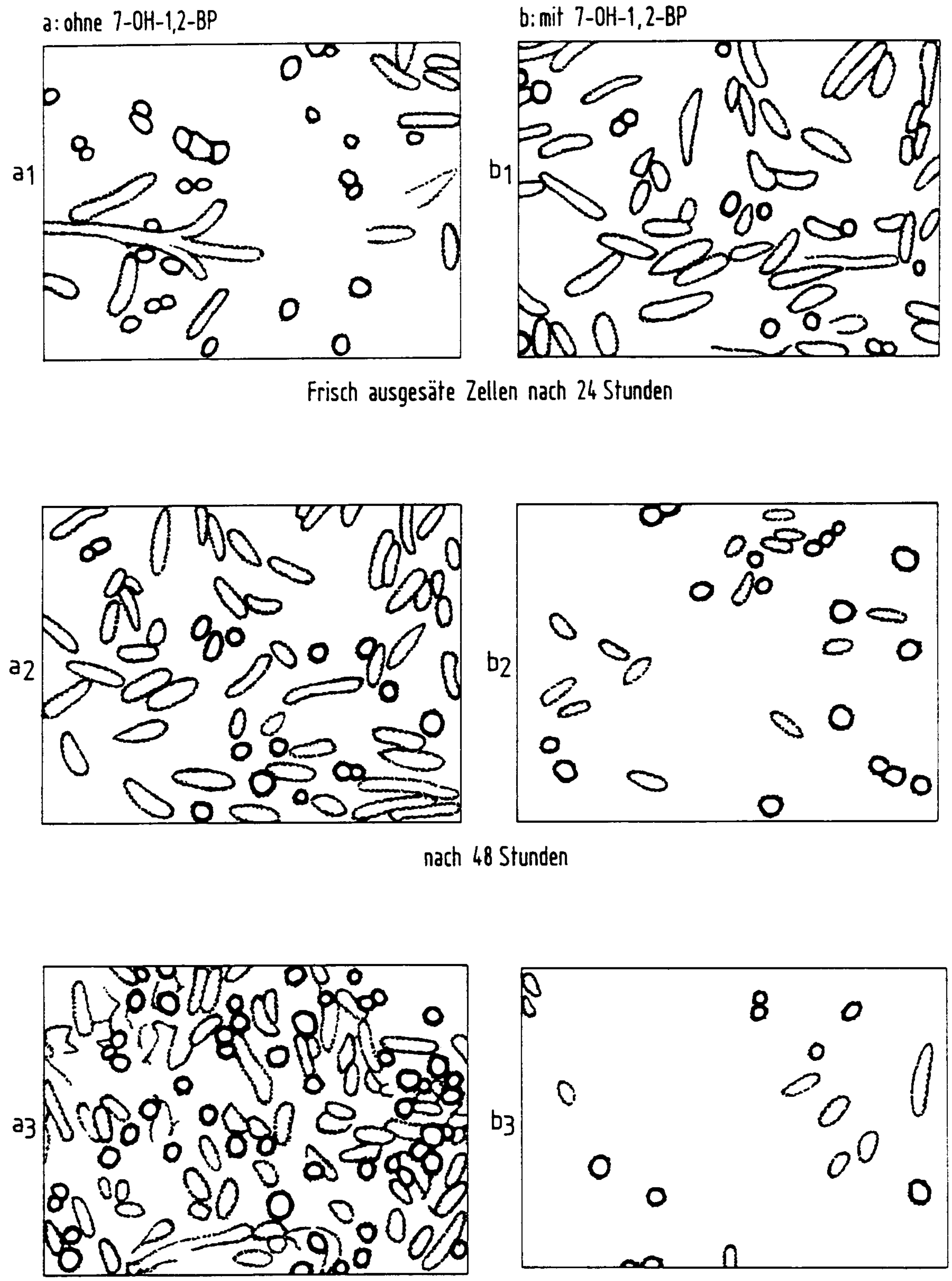


nach 72 Stunden

# Fig.11

DUKX-Zellen

a: ohne 7-OH-1,2-BP

b: mit 7-OH-1,2-BP

a1

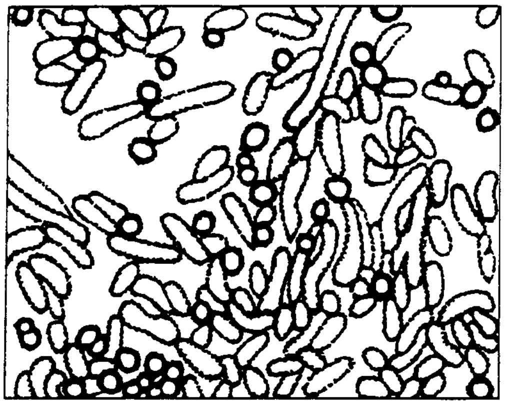

Frisch ausgesäte Zellen nach 72 Stunden

b1

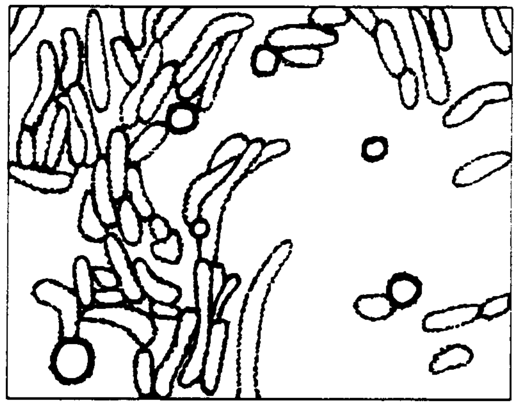

Zellen 24 Stunden in Gegenwart von 7-OH-1,2-BP angezogen, danach von Wirkstoff freigewaschen, Trypsinbehandelt, frisch ausgesät und 48 Stunden lang ohne 7-OH-1,2-BP kultiviert.

a2

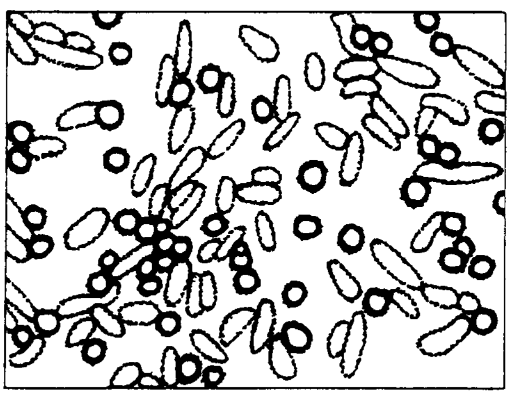

Frisch ausgesäte Zellen nach 72 Stunden

b2

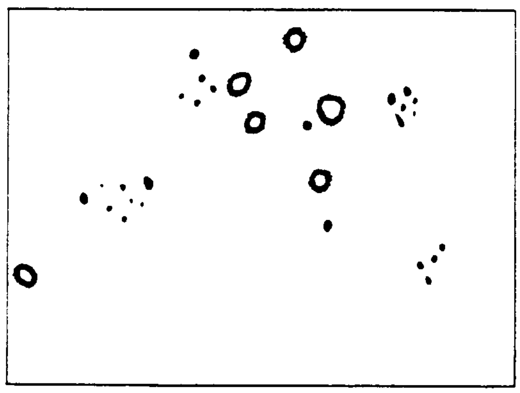

Zellen 24 Stunden lang in Gegenwart von 7-OH-1,2-BP angezogen, danach von Wirkstoff freigewaschen, Trypsinbehandelt, frisch ausgesät und 48 Stunden lang in Gegenwart von 7-OH-1,2BP kultiviert.

Fig.12

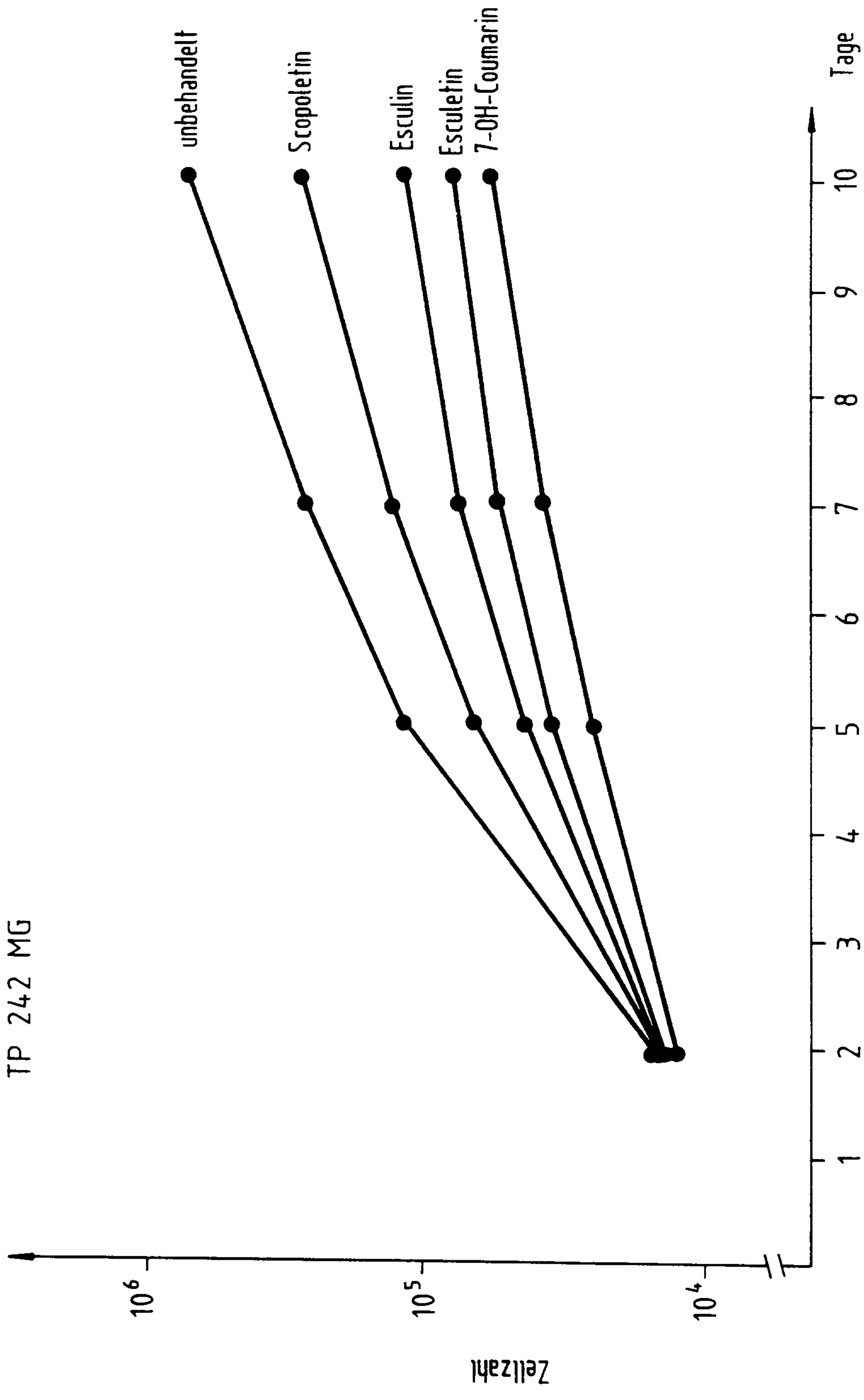
TP 242 MG
unbehandelt
Scopoletin
Esculin
Esculetin
7-OH-Coumarin
Tage
1
2
3
4
5
6
7
8
9
10
Zellzahl
$10^6$
$10^5$
$10^4$

Fig.13

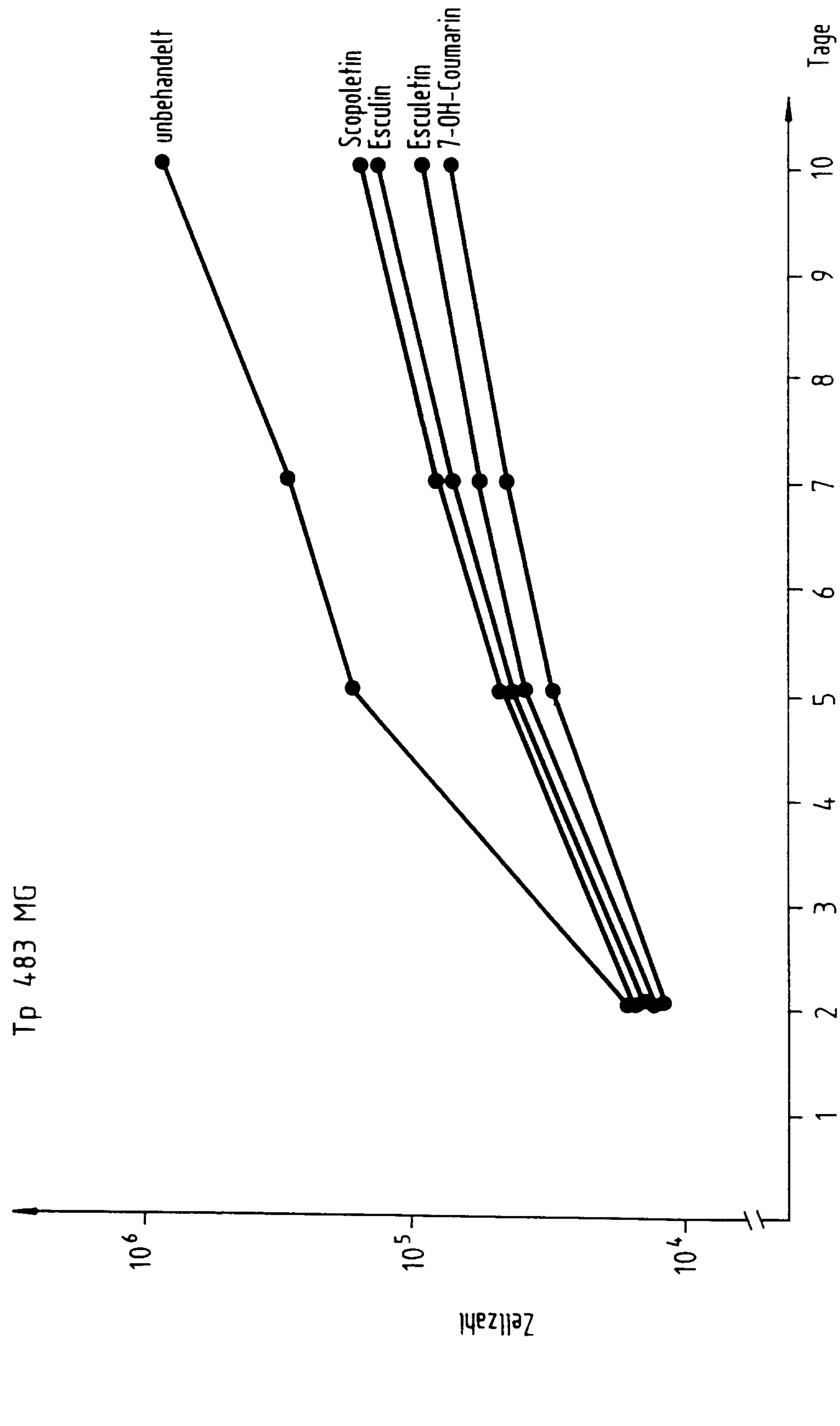
Tp 483 MG
unbehandelt
Scopoletin
Esculin
Esculetin
7-OH-Coumarin
Tage
1
2
3
4
5
6
7
8
9
10
$10^6$
$10^5$
$10^4$
Zellzahl

Fig.14

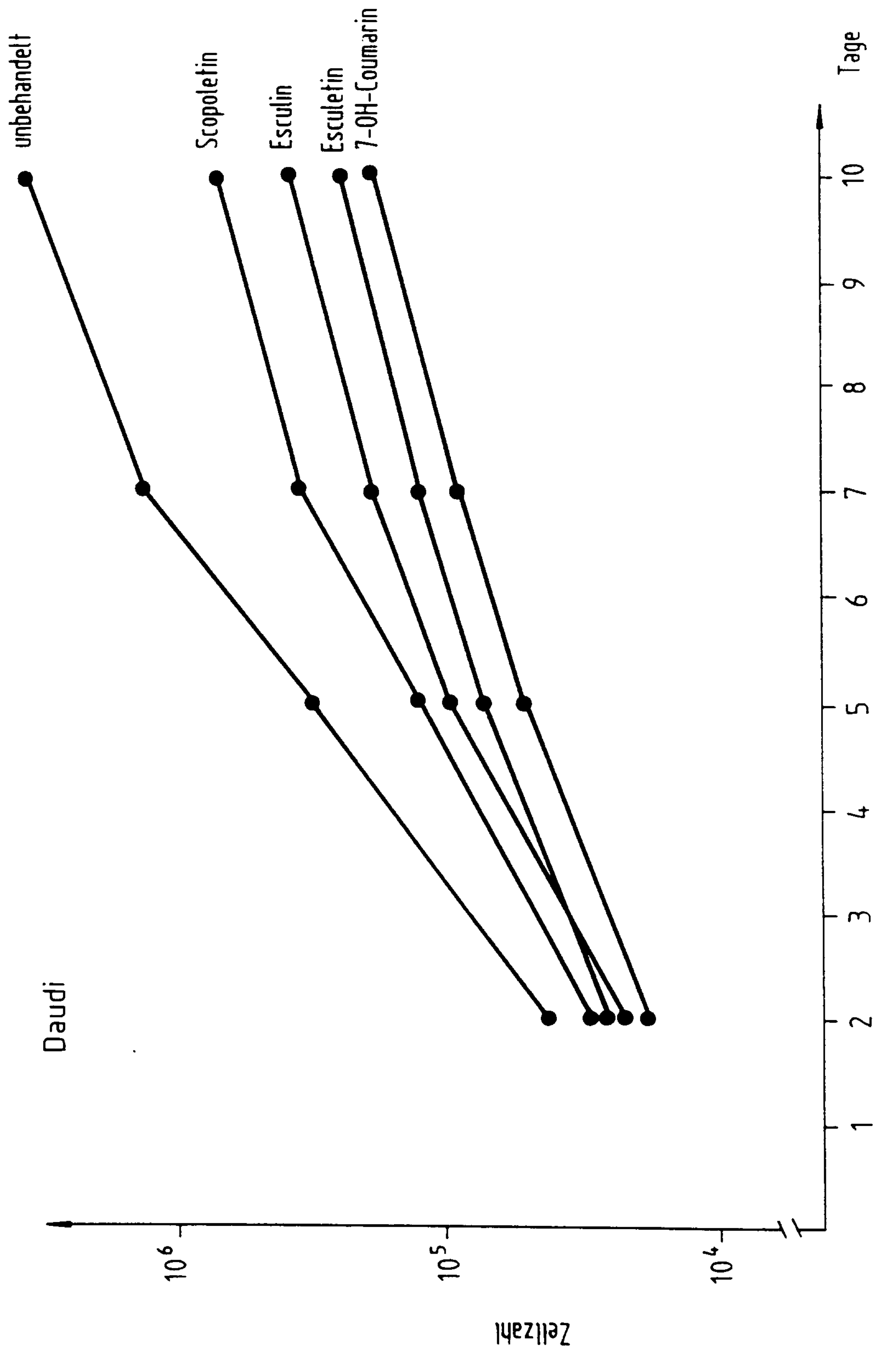
Daudi
unbehandelt
Scopoletin
Esculin
Esculetin
7-OH-Coumarin
Zellzahl
$10^6$
$10^5$
$10^4$
Tage
1
2
3
4
5
6
7
8
9
10

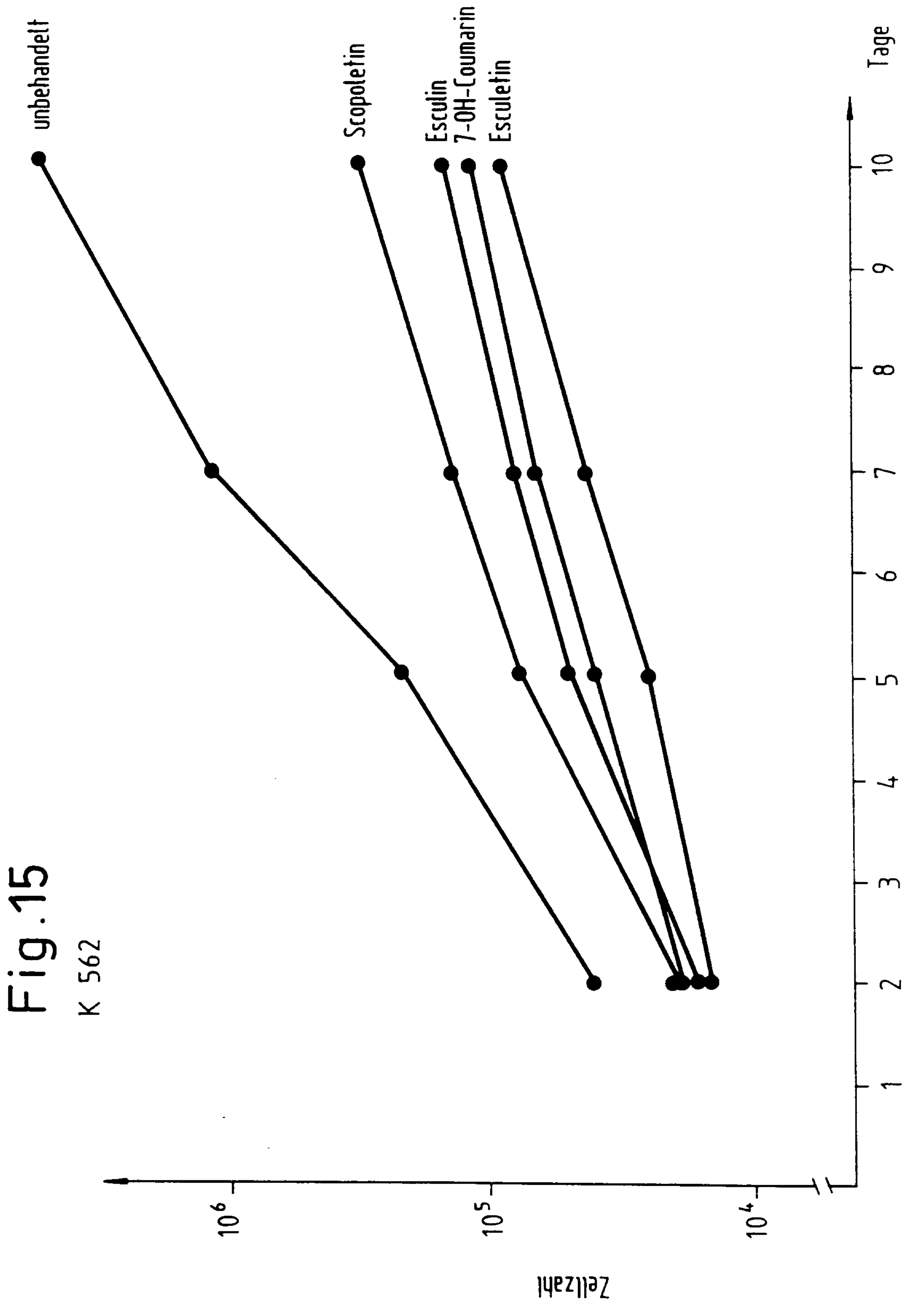
unbehandelt
Scopoletin
Esculin
7-OH-Coumarin
Esculetin
Tage
1
2
3
4
5
6
7
8
9
10
Zellzahl
$10^6$
$10^5$
$10^4$

Fig.15
K 562

Fig.16
CAK I-1

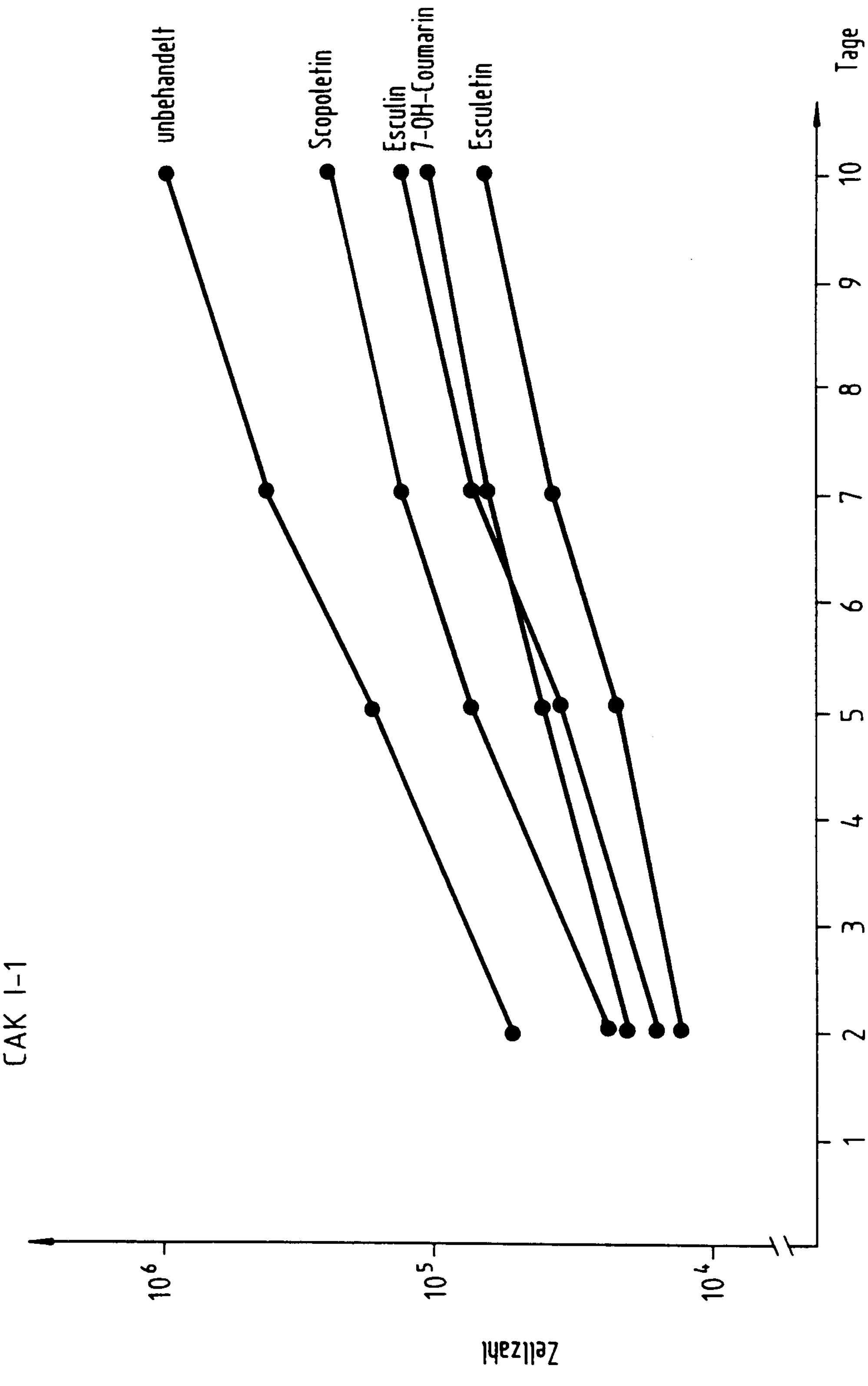
unbehandelt
Scopoletin
Esculin
7-OH-Coumarin
Esculetin
Tage
1
2
3
4
5
6
7
8
9
10
Zellzahl
$10^6$
$10^5$
$10^4$

Fig.17

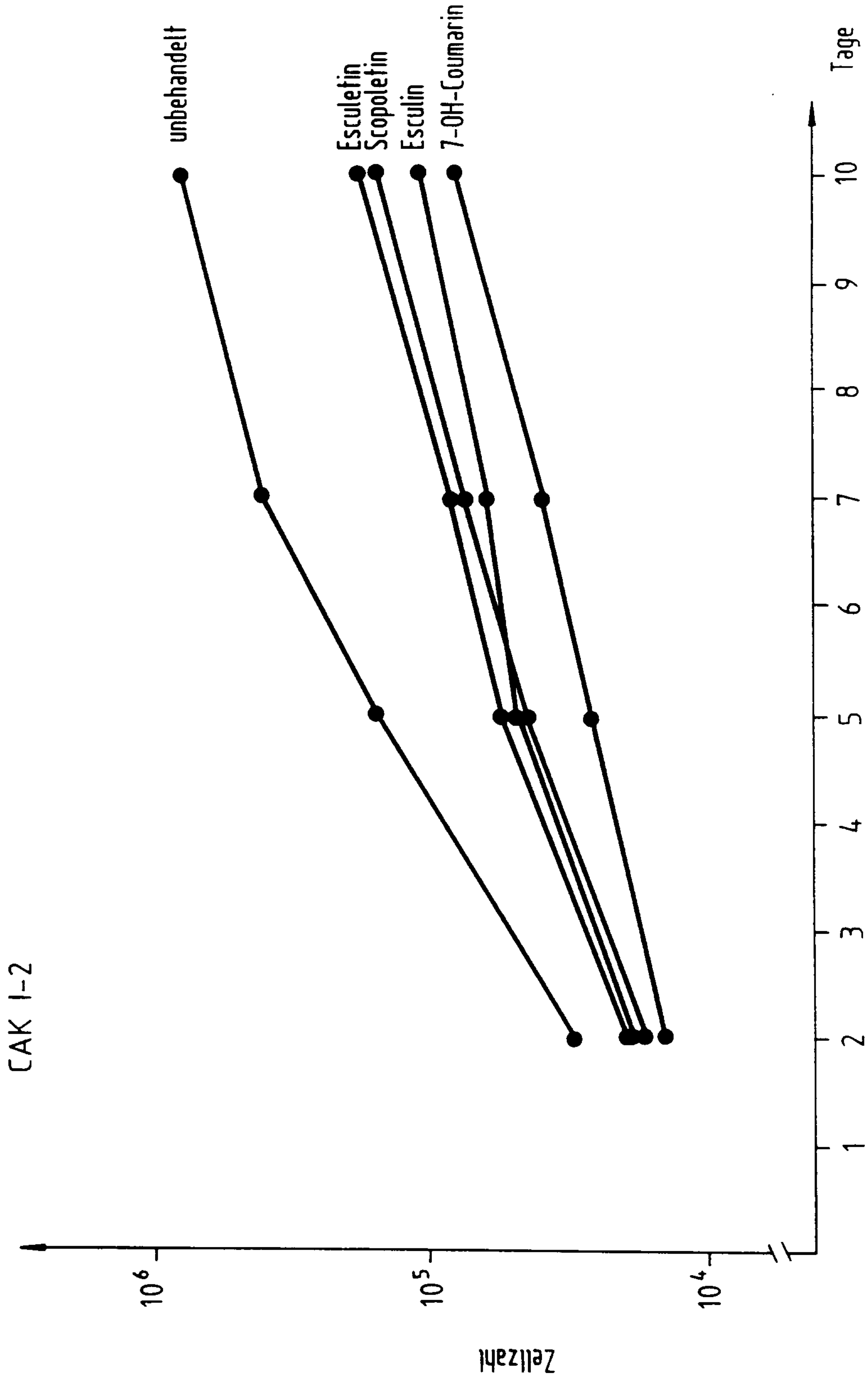
CAK I-2
Zellzahl
10^6
10^5
10^4
Tage
1
2
3
4
5
6
7
8
9
10
unbehandelt
Esculetin
Scopoletin
Esculin
7-OH-Coumarin

Fig. 18

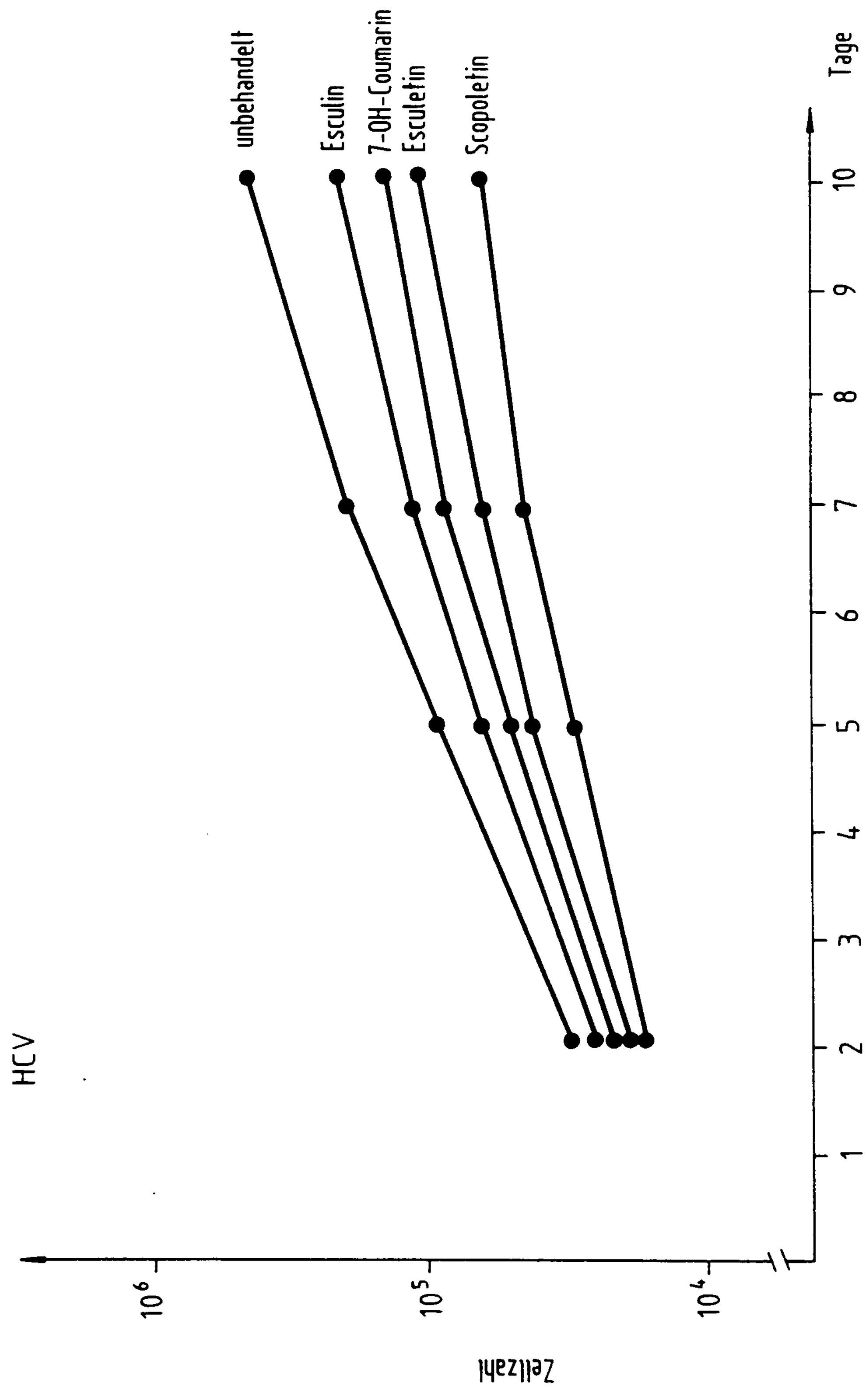
HCV
unbehandelt
Esculin
7-OH-Coumarin
Esculetin
Scopoletin
Tage
1
2
3
4
5
6
7
8
9
10
$10^6$
$10^5$
$10^4$
Zellzahl

Fig.19

MCF - 7

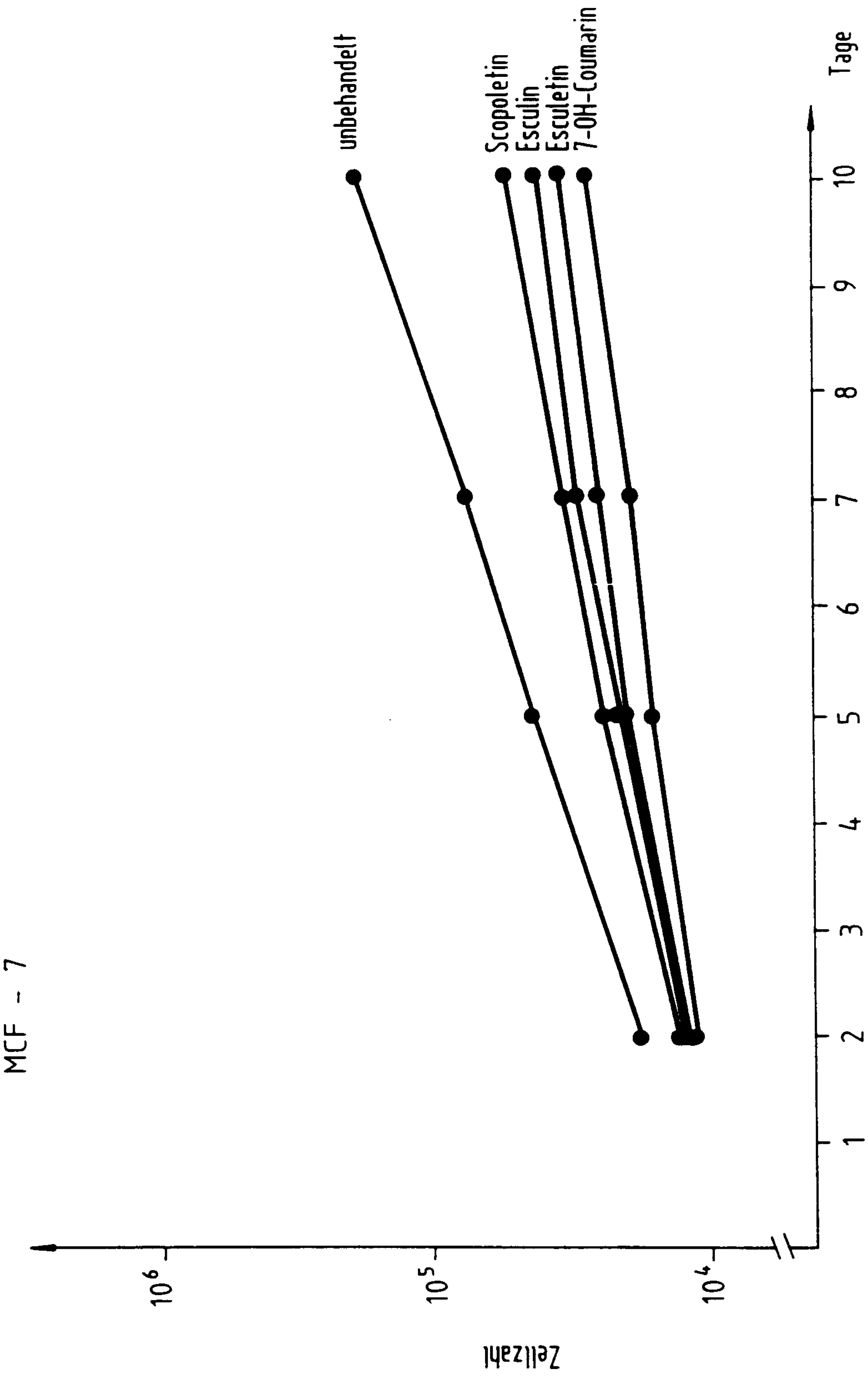
unbehandelt
Scopoletin
Esculin
Esculetin
7-OH-Coumarin
Tage
1
2
3
4
5
6
7
8
9
10
Zellzahl
$10^4$
$10^5$
$10^6$

Fig. 20

Z-R-75-1

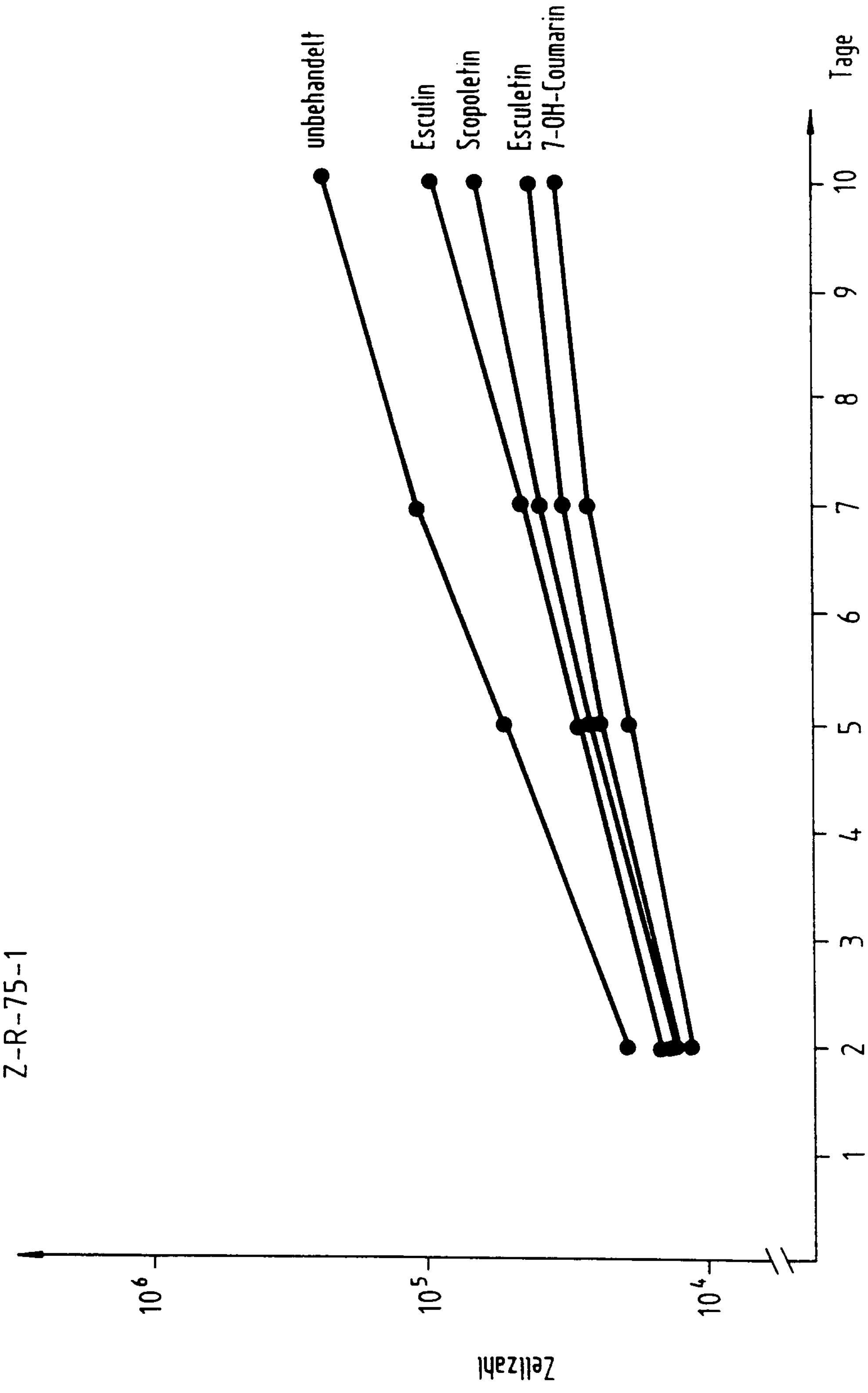
Zellzahl
$10^6$
$10^5$
$10^4$
Tage
1
2
3
4
5
6
7
8
9
10
unbehandelt
Esculin
Scopoletin
Esculetin
7-OH-Coumarin

Fig. 21

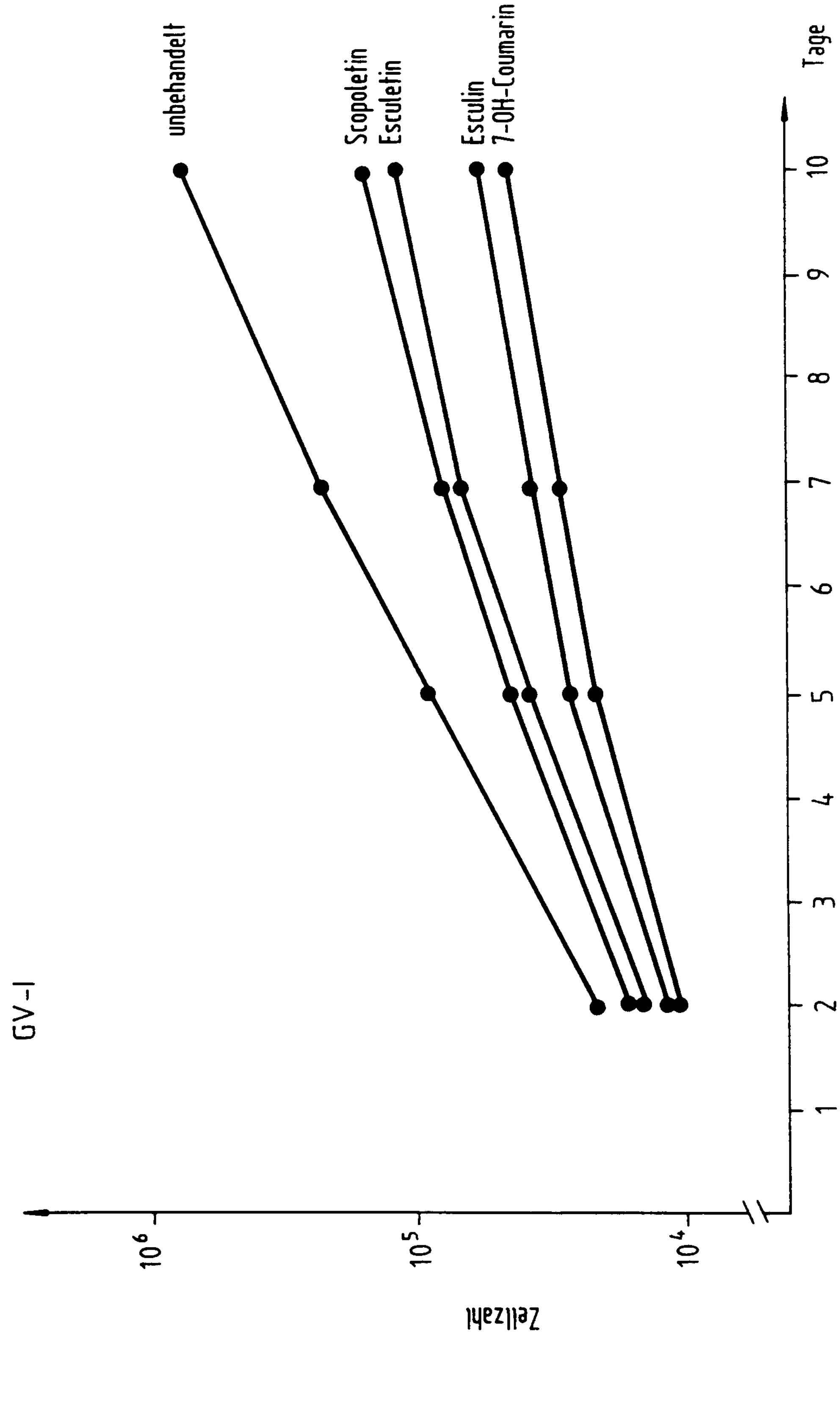
GV-I
unbehandelt
Scopoletin
Esculetin
Esculin
7-OH-Coumarin
Zellzahl
10⁶
10⁵
10⁴
Tage
1
2
3
4
5
6
7
8
9
10

Fig. 22
AtT 20

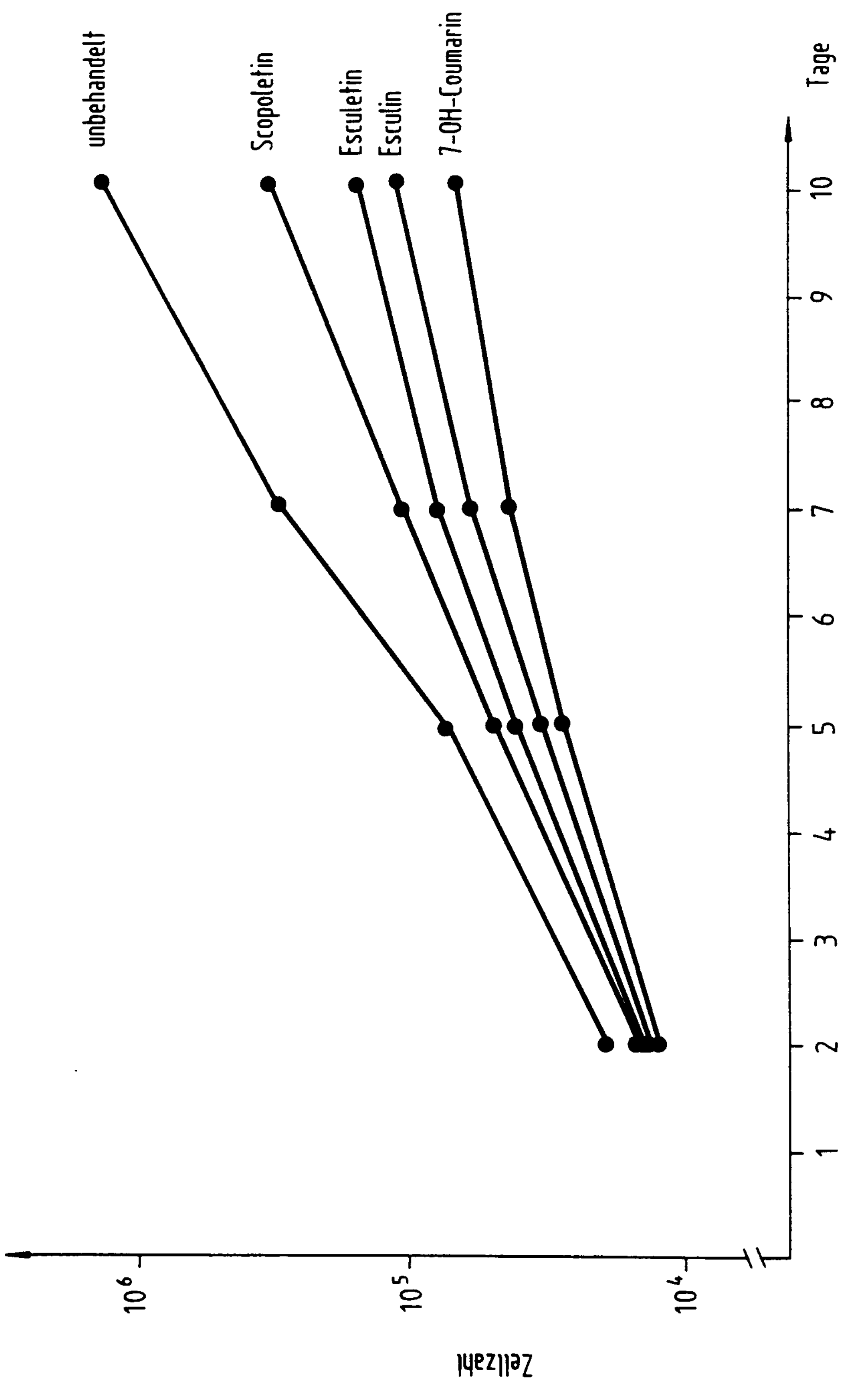
unbehandelt
Scopoletin
Esculetin
Esculin
7-OH-Coumarin
Tage
1
2
3
4
5
6
7
8
9
10
Zellzahl
$10^6$
$10^5$
$10^4$

Fig. 23

A - 375

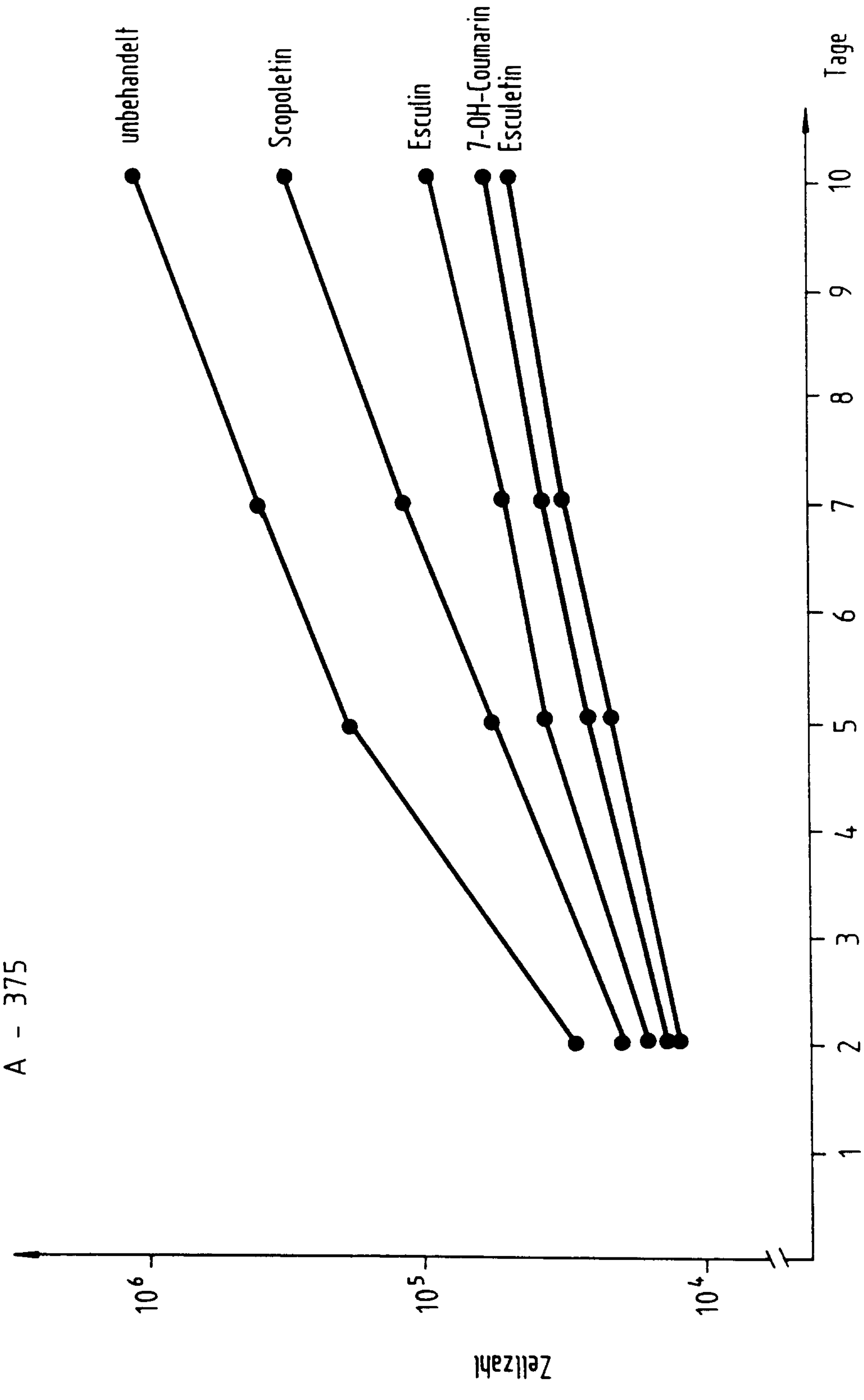

unbehandelt
Scopoletin
Esculin
7-OH-Coumarin
Esculetin
Tage
Zellzahl
$10^6$
$10^5$
$10^4$
1
2
3
4
5
6
7
8
9
10

Fig. 24

G - 361

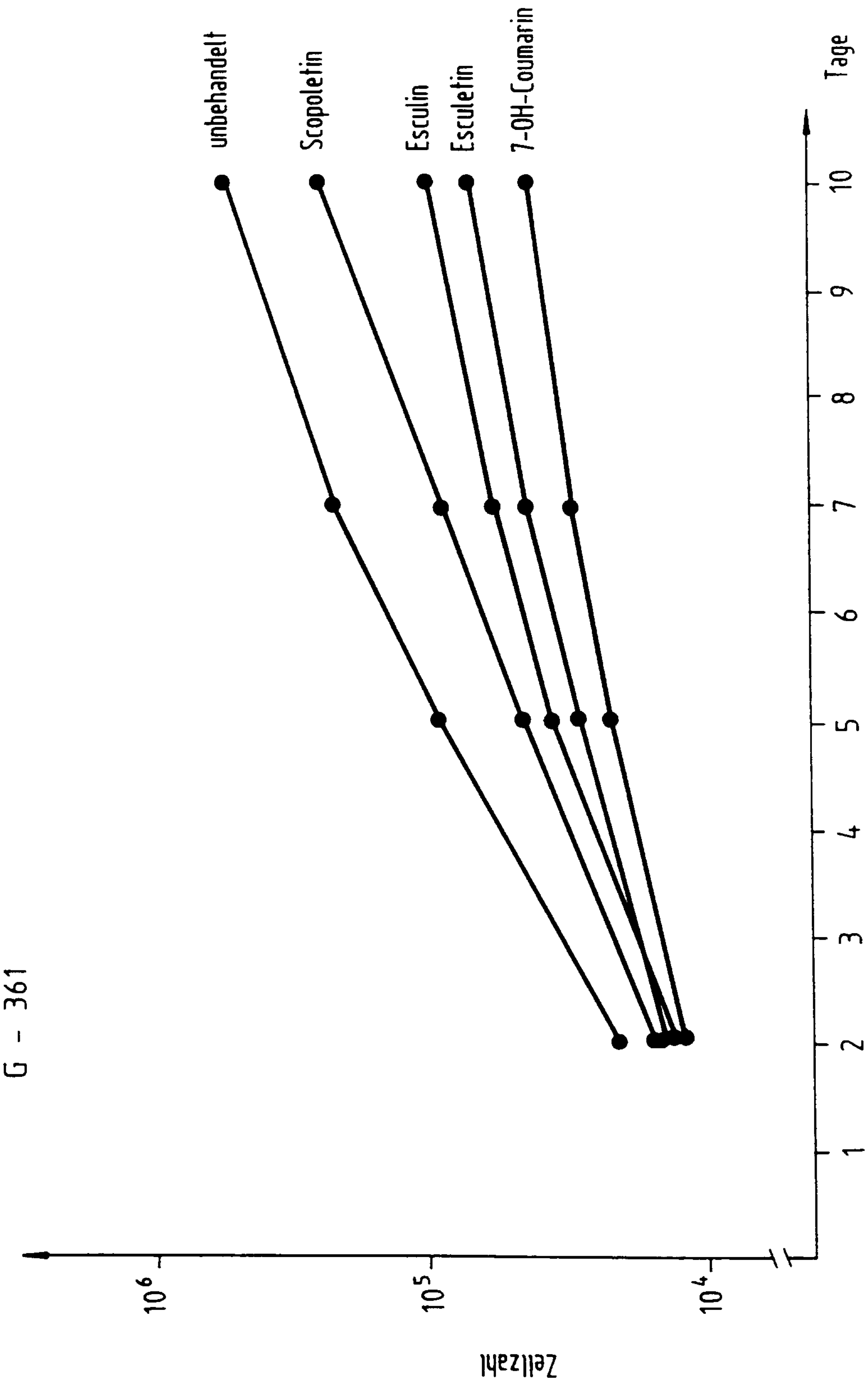
unbehandelt
Scopoletin
Esculin
Esculetin
7-OH-Coumarin
Tage
Zellzahl
$10^6$
$10^5$
$10^4$
1
2
3
4
5
6
7
8
9
10